# EUROPÄISCHE PATENTSCHRIFT

(11) **EP 0 355 552 B1**
(45) Veröffentlichungstag und Bekanntmachung des Hinweises auf die Patenterteilung: **23.02.1994**
(21) Anmeldenummer: 89114542.7
(22) Anmeldetag: 07.08.1989
(51) Int. Cl.: C09K 19/30, C07C 43/162

(54) **Alkoxymethyl- und Alkenyloxymethyl-cyclohexane**
Alkoxymethyl- and alkenyloxymethyl-cyclohexanes
Cyclohexanes alkoxyméthyls et alcényloxyméthyls

(30) Priorität: 15.08.1988 CH 3057/88; 18.05.1989 CH 1906/89
(43) Veröffentlichungstag der Anmeldung: 28.02.1990
(73) Patentinhaber: F. HOFFMANN-LA ROCHE AG, 4002 Basel (CH)
(72) Erfinder: Buchecker, Richard, Dr., CH-8008 Zürich (CH); Schadt, Martin, Dr., CH-4411 Seltisberg (CH); Villiger, Alois, Dr., CH-4055 Basel (CH)
(74) Vertreter: Cottong, Norbert A.

(56) Entgegenhaltungen:
- EP-A- 0 090 548
- EP-A- 0 122 389
- EP-A- 0 132 553
- EP-A- 0 361 532
- PATENT ABSTRACTS OF JAPAN Band 8, Nr.212, 27.September 1984; & JP-A-59 98 053

## Beschreibung

Die vorliegende Erfindung betrifft neue Alkoxymethyl-und Alkenyloxymethylcyclohexane, deren Herstellung, flüssigkristalline Gemische, die diese Verbindungen enthalten, sowie die Verwendung für elektro-optische Zwecke.

Flüssige Kristalle werden vor allem als Dielektrika in Anzeigevorrichtungen verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirk-Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben. Weiterhin sollten sie bei üblichen Betriebstemperturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielweise sollten Materialien für Zellen mit verdrillt nematischer Struktur eine positive dielektrische Anisotropie und eine möglichst geringe elektrische Leitfähigkeit aufweisen.

In letzter Zeit besteht ein vermehrtes Interesse an Materialien mit niedriger optischer Anisotropie, insbesondere für aktiv adressierte Flüssigkristallanzeigen, wie MIM-Anwendungen (Metall-Isolator-Metall) oder TFT-Anwendungen ("thin film transistor" = Dünnfilmtransistor) in Fernsehgeräten. Verbindungen mit niedriger optischer Anisotropie sind beispielsweise die in EP-A-0 132 553 und in EP-A-0 090 548 beschriebenen Bicyclohexyl-Derivate mit Alkyl/Alkoxyalkyl Seitenketten. Der Stand der Technik nach Artikel 54(3)EPÜ offenbart in EP-A-361 532 obenfalls Bicyclohexyl-Derivate mit Alkyl/Alkoxy Seitenketten. Die bekannten flüssigkristallinen Verbindungen mit niedriger optischer Anisotropie erzeugen jedoch in Mischungen häufig smektische Tendenzen und führen meist zu einer unerwünschten Erhöhung der Schwellenspannung, der Viskosität und/oder der Ansprechzeiten.

Gegenstand der Erfindung sind die Verbindungen der allgemeinen Formel

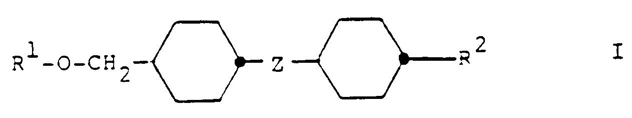

worin Z eine einfache Kovalenzbindung oder -CH₂CH₂-darstellt; R¹ Alkyl oder 2-Alkenyl bezeichnet; und R² 1E-Alkenyl, 3E-Alkenyl, 4-Alkenyl, (2-Alkenyl)oxymethyl oder, wenn R¹ 2-Alkenyl bezeichnet und Z -CH₂CH₂- darstellt, auch Alkyl oder Alkoxymethyl bedeutet.

Die erfindungsgemässen Verbindungen sind Flüssigkristalle mit sehr niedriger optischer Anisotropie, relativ tiefen Schmelzpunkten und meist vergleichsweise hohen Klärpunkten. Sie sind in bekannten Flüssigkristallmaterialien sehr gut löslich und ergeben in Mischungen ein gutes Schmelzverhalten.

Die erfindungsgemässen Verbindungen besitzen niedrige Viskositäten und ergeben in Mischungen erstaunlich kurze Schaltzeiten. Insbesondere sind die Einschaltzeiten in der Regel erheblich kürzer als diejenigen von Mischungen vergleichbarer Verbindungen mit niedriger optischer Anisotropie.

Die kurzen Ansprechzeiten und das gute Schmelzverhalten lassen sich auch in komplexe Mischungen so übertragen, dass rasch schaltende Flüssigkristalle mit hohen Klärpunkten. tiefen Schmelzpunkten und gleichzeitig tiefen Schwellenspannungen, kurzen Ansprechzeiten (insbesondere bei tiefen Temperaturen) und vergleichsweise niedrigen optischen Anisotropien erhalten werden.

Je nach Bedeutung von R¹ und R² können diese Eigenschaften bis zu einem gewissen Grad variiert werden. 4-Alkenyl oder (2-Alkenyl)oxymethyl in R² und/oder 2-Alkenyl in R¹ führt beispielsweise zu besonders niedrigen Schwellenspannungen und optischen Anisotropien. Verbindungen der Formel I, worin R² 1E-Alkenyl oder 3E-Alkenyl bedeutet, ergeben hingegen in der Regel höhere Klärpunkte und kürzere Ansprechzeiten.

Die Verbindungen der Formel I sind unpolare Verbindungen mit kleinem Absolutwert der dielektrischen Anisotropie.

Die erfindungsgemässen Verbindungen eignen sich insbesondere als Komponenten nematischer und cholesterischer Gemische. Sie können in den üblichen elektro-optischen Vorrichtungen verwendet werden, beispielsweise in den eingangs erwähnten Flüssigkristallzellen. Aufgrund der niedrigen optischen Anisotropie eignen sie sich insbesondere zum Einsatz in Flüssigkristallmischungen für OMI-Zellen, für TFT-Anwendungen, für TN-Zellen, die im ersten Minimum betrieben werden, und dergleichen.

Formel I umfasst die Verbindungen der allgemeinen Formeln 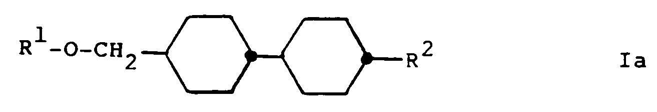
worin R¹ Alkyl und R² 1E-Alkenyl, 3E-Alkenyl, 4-Alkenyl oder (2-Alkenyl)oxymethyl bezeichnen, oder R¹ 2-Alkenyl und R² 1E-Alkenyl, 3E-Alkenyl, 4-Alkenyl, (2-Alkenyl)oxymethyl, Alkyl oder Alkoxymethyl bezeichnen,
und 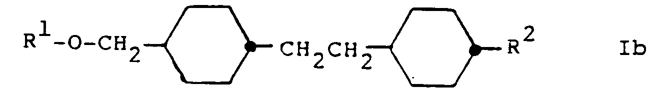
worin R¹ Alkyl und R² 1E-Alkenyl, 3E-Alkenyl, 4-Alkenyl, (2-Alkenyl)oxymethyl, Alkyl oder Alkoxymethyl bezeichnen, oder R¹ 2-Alkenyl und R² 1E-Alkenyl, 3E-Alkenyl, 4-Alkenyl, (2-Alkenyl)oxymethyl, Alkyl oder Alkoxymethyl bezeichnen.

Die Ausdrücke "Alkyl", "1E-Alkenyl", "2-Alkenyl", "3E-Alkenyl", "4-Alkenyl", "Alkoxymethyl" und "(2-Alkenyl)oxymethyl" umfassen im Rahmen der vorliegenden Erfindung geradkettige und verzweigte Gruppen. Beispiele bevorzugter Gruppen sind Methyl, Aethyl, Propyl, Butyl (=n-Butyl), s-Butyl, 2-Methylbutyl, Pentyl (=n-Pentyl), 2-Pentyl, Hexyl, Heptyl, Vinyl, 1E-Propenyl, 1E-Butenyl, 1E-Pentenyl, 1E-Hexenyl, 1E-Heptenyl, Allyl, 2E-Butenyl, 2Z-Butenyl, 2E-Pentenyl, 2Z-Pentenyl, 3-Butenyl, 3E-Pentenyl, 3E-Hexenyl, 3E-Heptenyl, 4-Pentenyl, 4E-Hexenyl, 4Z-Hexenyl, 4E-Heptenyl, 4Z-Heptenyl, Methoxymethyl, Aethoxymethyl, Propyloxymethyl, Butyloxymethyl, s-Butyloxymethyl, Pentyloxymethyl, (2-Pentyl)oxymethyl, (2-Methylbutyl)oxymethyl, Allyloxymethyl, (2E-Butenyl)oxymethyl, (2Z-Butenyl)oxymethyl, (2E-Pentenyl)oxymethyl, (2Z-Pentenyl)oxymethyl und dergleichen. 2-Alkenyl und 4-Alkenylgruppen können in E-Konfiguration oder vorzugsweise in Z-Konfiguration vorliegen.

Verbindungen der Formel I mit geradkettigen Resten R¹ und R² sind im allgemeinen bevorzugt. Gewünschtenfalls kann jedoch R¹ und/oder R² verzeigt sein, wobei verzweigte Gruppen vorzugsweise chiral sein können. Optisch aktive Verbindungen der Formel I mit chiralem R¹ und/oder R² eignen sich beispielsweise als optisch aktive Dotierstoffe zur Herstellung cholesterischer Gemische oder zur Vermeidung der Umkehr der Verdrillungsrichtung (reverse twist) in TN-Zellen.

R¹ in obigen Formeln I, Ia und Ib besitzt vorzugsweise höchstens 10 Kohlenstoffatome, besonders bevorzugt höchstens 5 Kohlenstoffatome. Bevorzugte Reste R¹ sind somit C₁-C₁₀-Alkyl und C₃-C₁₀-2-Alkenyl, insbesondere C₁-C₅-Alkyl und C₃-C₅-2-Alkenyl. Beispiele besonders bevorzugter Reste R¹ sind Methyl, Aethyl, Propyl, Allyl und 2-Butenyl.

R² in obigen Formeln I, Ia und Ib besitzt vorzugsweise höchstens 12 Kohlenstoffatome, besonders bevorzugt höchstens 7 Kohlenstoffatome. Bevorzugte Reste R² sind somit C₂-C₁₂-1E-Alkenyl, C₄-C₁₂-3E-Alkenyl, C₅-C₁₂-4-Alkenyl, C₄-C₁₂-(2-Alkenyl)oxymethyl, C₁-C₁₂-Alkyl und C₂-C₁₂-Alkoxymethyl, insbesondere C₂-C₇-1E-Alkenyl, C₄-C₇-3E-Alkenyl, C₅-C₇-4-Alkenyl, C₄-C₇-(2-Alkenyl)oxymethyl, C₁-C₇-Alkyl und C₂-C₇-Alkoxymethyl. Beispiele besonders bevorzugter Reste R² sind Vinyl, 1E-Propenyl, 1E-Butenyl, 1E-Pentenyl, 3-Butenyl, 3E-Pentenyl, 4-Pentenyl, Allyloxymethyl, (2-Butenyl)oxymethyl, Methyl, Aethyl, Propyl, Butyl, Pentyl, Methoxymethyl, Aethoxymethyl, Propyloxymethyl und Butyloxymethyl.

Verbindungen der Formel I, welche in R¹ und/oder R² eine C-C-Doppelbindung aufweisen sind im allgemeinen bevorzugt. Vorzugsweise steht somit in obigen Formeln I, Ia und Ib R¹ für 2-Alkenyl und/oder R² für 1E-Alkenyl, 3E-Alkenyl, 4-Alkenyl oder (2-Alkenyl)oxymethyl.

Die Verbindungen der Formel I können erfindungsgemäss dadurch hergestellt werden, dass man eine Verbindung der allgemeinen Formel 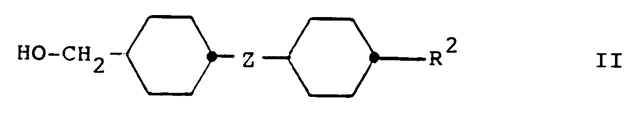
mit einer Verbindung der allgemeinen Formel

R¹-X III

worin X Chlor, Brom oder Jod bezeichnet, und R¹ und R² die obigen Bedeutungen haben,
veräthert.

Die Verätherung kann in an sich bekannter Weise unter Verwendung üblicher Basen erfolgen. Vorzugsweise erfolgt die Verätherung mit dem Bromid oder Jodid der Formel III.

Die Verbindungen der Formel II können in an sich bekannter Weise aus den Nitrilen der allgemeinen Formel 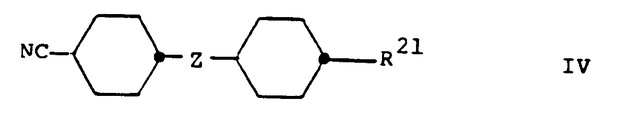
worin Z eine einfache Kovalenzbindung oder -CH₂CH₂-darstellt, und R²¹ 1E-Alkenyl, 3E-Alkenyl, 4-Alkenyl, (2-Alkenyl)oxymethyl, Alkyl oder Alkoxymethyl bedeutet,
hergestellt werden, beispielsweise durch Hydrolyse der Cyanogruppe und anschliessende Reduktion der Carboxygruppe zur Hydroxymethylgruppe. Die Hydrolyse und Reduktion können vorzugsweise nach den in den Synthesebeispielen beschriebenen Methoden erfolgen.

Die Verbindungen der Formel IV, worin Z eine einfache Kovalenzbindung und R²¹ (2-Alkenyl)oxymethyl bedeuten oder Z -CH₂CH₂- und R²¹ Alkoxymethyl oder (2-Alkenyl)oxymethyl bedeuten, sind neue Zwischenprodukte und können auf an sich bekannte Weise hergestellt werden.

Die Verbindungen der Formel I können in Form von Gemischen untereinander und/oder mit anderen Flüssigkristallkomponenten verwendet werden, wie z.B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Phenylcyclohexane, Cyclohexylcyclohexane, Phenylpyrimidine, Cyclohexylpyrimidine, Phenyldioxane, 2-Cyclohexyl-1-phenyläthane, Terphenyle, Cyclohexylbiphenyle, Cyclohexylphenylpyrimidine und dergleichen. Derartige Substanzen sind dem Fachmann bekannt und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I und/oder andere Flüssigkristallkomponenten sein.

Die Verbindungen der Formel I eignen sich insbesondere für nematische oder, sofern mindestens eine Komponente des Gemisches optisch aktiv ist, auch für cholesterische Gemische. Ein bevorzugter Anwendungsbereich betrifft die Verwendung als Dielektrikum in Flüssigkristallanzeigevorrichtungen mit verdrillt nematischer Flüssigkristallstruktur, wie TN-Zellen, STN-Zellen, SBE-Zellen und OMI-Zellen. Bevorzugte Gemische sind daher diejenigen, welche eine oder mehrere Verbindungen der Formel I und eine oder mehrere Verbindungen mit positiver dielektrischer Anisotropie enthalten.

Aufgrund der guten Löslichkeit der Verbindungen der Formel I in anderen Flüssigkristallmaterialien und aufgrund ihrer guten Mischbarkeit untereinander kann der Anteil der Verbindungen der Formel I in den erfindungsgemässen Gemischen relativ hoch sein und beispielsweise etwa 1-70 Gew.-% betragen. Im allgemeinen ist ein Anteil von etwa 3-40 Gew.-%, insbesondere etwa 5-30 Gew.-% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln

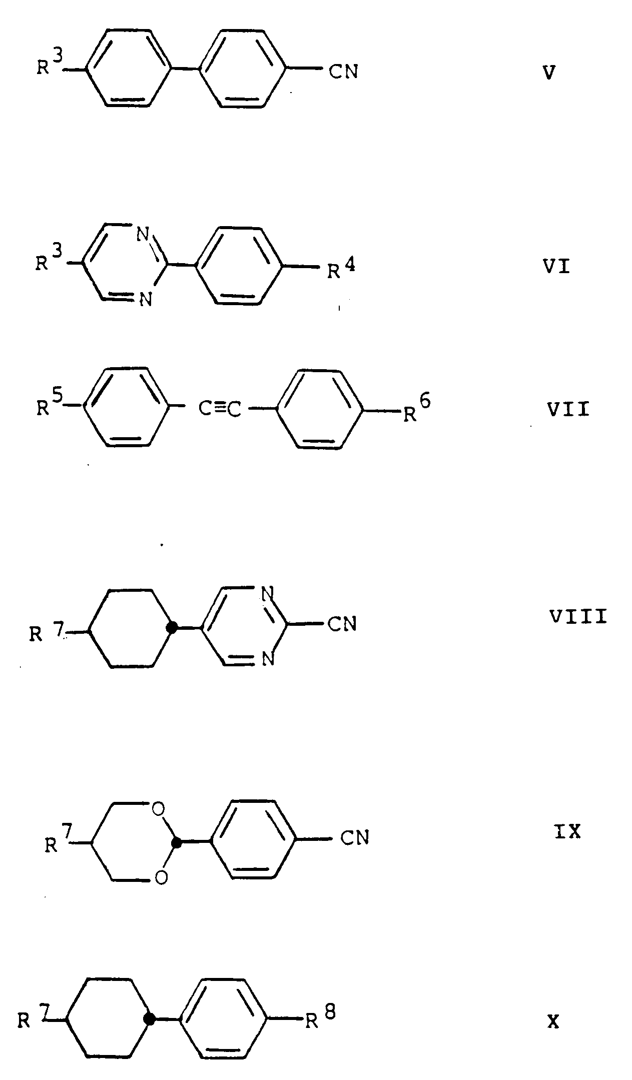

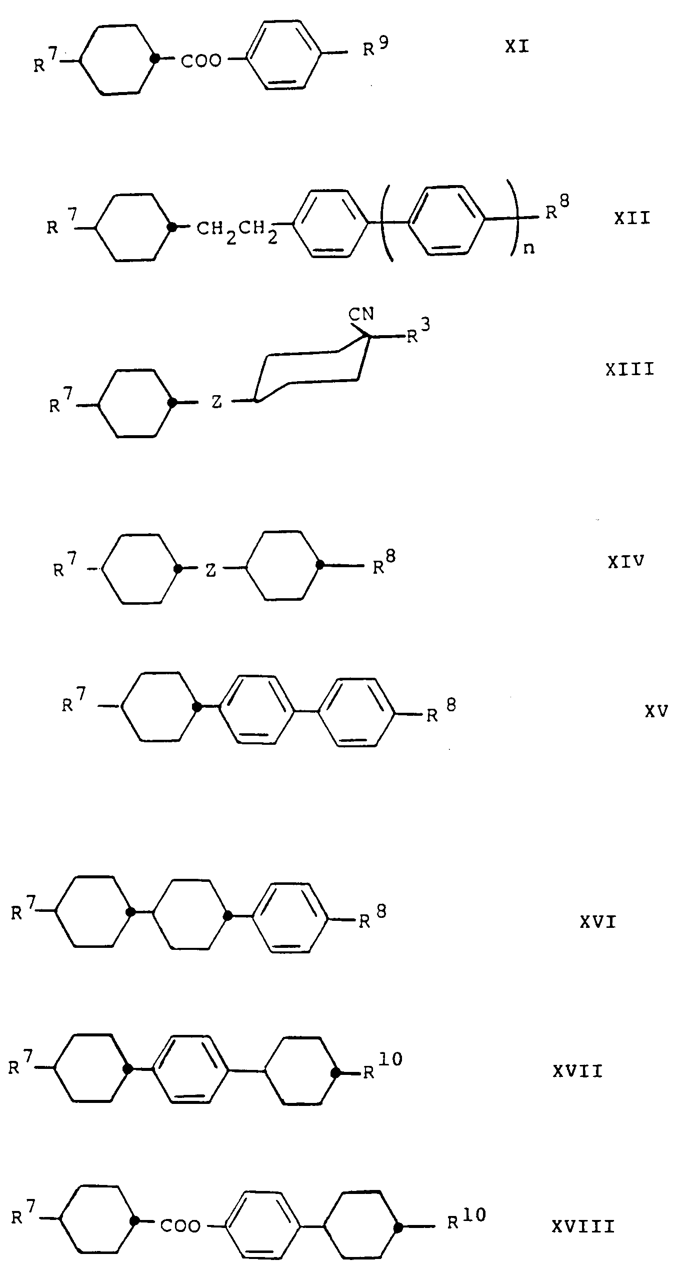

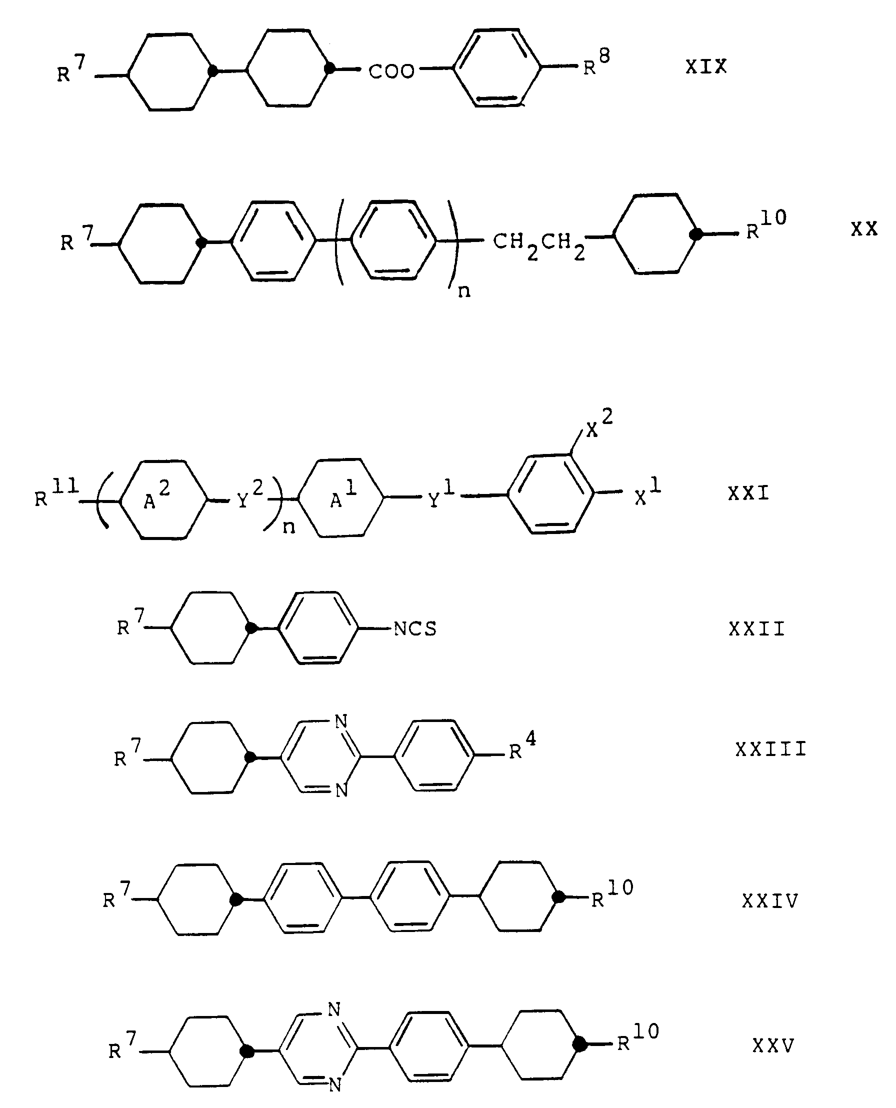

worin R³ Alkyl, 3E-Alkenyl oder 4-Alkenyl bedeutet; R⁴ Alkyl, Cyano oder Fluor darstellt; R⁵ und R⁶ Alkyl oder Alkoxy bezeichnen; R⁷ und R¹⁰ unabhängig voneinander Alkyl, 1E-Alkenyl, 3E-Alkenyl oder 4-Alkenyl bedeuten; R⁸ Cyano, Alkyl, 1E-Alkenyl, 3E-Alkenyl, 4-Alkenyl, Alkoxy, 2E-Alkenyloxy oder 3-Alkenyloxy bezeichnet; R⁹ Alkoxy, 2E-Alkenyloxy oder 3-Alkenyloxy bedeutet; n für die Zahl 0 oder 1 steht; Z eine einfache Kovalenzbindung oder -CH₂CH₂- darstellt; X¹ Fluor oder Chlor und X² Wasserstoff, Fluor oder Chlor bezeichnen; R¹¹ Alkyl, 3E-Alkenyl, 4-Alkenyl, Alkoxy, 2E-Alkenyloxy oder 3-Alkenyloxy bedeutet; eine der Gruppen Y¹ und Y² eine einfache Kovalenzbindung, -COO-, -OOC-, -CH₂CH₂-, -CH₂O- oder -OCH₂- und die andere der Gruppen Y¹ und Y² eine einfache Kovalenzbindung bedeutet; und die Ringe A¹ und A² unabhängig voneinander substituiertes oder unsubstituiertes trans-1,4-Cyclohexylen, in welchem gegebenenfalls 2 nicht benachbarte CH₂-Gruppen durch Sauerstoff ersetzt sind, oder substituiertes oder unsubstituiertes 1,4-Phenylen, in welchem gegebenenfalls 1 oder 2 CH₂-Gruppen durch Stickstoff ersetzt sind, darstellen.

Der Ausdruck "substituiertes oder unsubstituiertes trans-1,4-Cyclohexylen, in welchem gegebenenfalls 2 nicht benachbarte CH₂-Gruppen durch Sauerstoff ersetzt sind", umfasst insbesondere trans-1,4-Cyclohexylen und trans-m-Dioxan-2,5-diyl sowie Ringe, die mit in Flüssigkristallen üblichen Substituenten, wie Cyano, Methyl, Fluor oder Chlor, substituiert sind, beispielsweise 1-Cyano-trans-1,4-cyclohexylen oder 2-Methyl-trans-1,4-cyclohexylen.

Der Ausdruck "substituiertes oder unsubstituiertes 1,4-Phenylen, in welchem gegebenenfalls 1 oder 2 CH-Gruppen durch Stickstoff ersetzt sind", umfasst insbesondere 1,4-Phenylen, Pyridin-2,5-diyl, Pyrazin-2,5-diyl und Pyrimidin-2,5-diyl, sowie Ringe, die mit in Flüssigkristallen üblichen Substituenten, wie Cyano, Methyl, Fluor oder Chlor substituiert sind, beispielsweise 2-Cyano-1,4-phenylen, 2-Fluor-1,4-phenylen, 2-Chlor-1,4-phenylen oder 2-Methyl-1,4-phenylen.

Bevorzugte Mischungskomponenten mit positiver dielektrischer Anisotropie sind die Cyano- und Halogenverbindungen der Formeln V, VI, VIII, IX, X, XII, XIV, XV, XIX, XXI und XXIII sowie die Isothiocyanate der Formel XXII. Vorzugsweise enthält das Gesamtgemisch etwa 20-70 Gew.-%, insbesondere etwa 25-50 Gew.-% an einer oder mehreren dieser Verbindungen.

Bevorzugte Mischungskomponenten zur Erzielung einer niedrigen optischen Anisotropie im Gesamtgemisch sind die Verbindungen der Formeln VIII-XXI, insbesondere die Verbindungen der Formeln XIII, XIV, XVI, XVII, XVIII, XIX und XX sowie die Verbindungen der Formel XXI, worin die Ringe A¹ und A² trans-1,4-Cyclohexylen bedeuten.

Besonders bevorzugt sind im allgemeinen diejenigen Gemische, welche neben einer oder mehreren Verbindungen der Formel I eine oder mehrere Verbindungen der Formel VIII, X, XIV, XVI, XVII und/oder XXI enthalten.

Die erfindungsgemässen Mischungen können ferner optisch aktive Verbindungen (z.B. optisch aktive 4′-Alkyl- oder 4′-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe, Farbe, Extinktion und dergleichen bestimmt. Im allgemeinen beträgt der Anteil optisch aktiver Verbindungen und dichroitischer Farbstoffe höchstens je etwa 10 Gew.-% im Gesamtgemisch.

Die Herstellung der erfindungsgemässen Gemisch und die Herstellung der elektro-optischen Vorrichtungen können in an sich bekannter Weise erfolgen.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische, S_{B} eine smektisch B, N eine nematische und I die isotrope Phase. V₁₀ bezeichnet die Spannung für 10% Transmission (Blickrichtung senkrecht zur Plattenoberfläche). tₒₙ und t_{off} bezeichnen die Einschaltzeit bzw. die Ausschaltzeit und Δn bezeichnet die optische Anisotropie.

### Beispiel 1

a) Eine Lösung von 1,04 g trans-4-[trans-4-(3-Butenyl)cyclohexyl]cyclohexancarbonitril in 10 ml Diäthylenglykol wurde mit einer Lösung von 2 g Kaliumhydroxid in 10 ml Diäthylenglykol versetzt und dann 2,5 Stunden bei 180°C (Oelbadtemperatur) gerührt. Anschliessend wurde das Reaktionsgemisch auf Eis/Wasser gegossen, mit 8 ml 25-prozentiger Salzsäure angesäuert und mit Diäthyläther extrahiert. Die organische Phase wurde über Magnesiumsulfat getrocknet, filtriert und eingedampft. Kristallisation des erhaltenen beigen Rohproduktes (1,06 g) aus 50 ml Hexan ergab 588 mg trans-4-[trans-4-(3-Butenyl)cyclohexyl]cyclohexancarbonsäure als farblose Kristalle.
b) Eine Suspension von 1,55 g Lithiumaluminiumhydrid in 30 ml Diäthyläther wurde bei Raumtemperatur innert 10 Minuten tropfenweise mit einer Lösung von 2,7 g trans-4-[trans-4-(3-Butenyl)cyclohexyl]cyclohexancarbonsäure in 40 ml Diäthyläther versetzt. Anschliessend wurde das Reaktionsgemisch 1 Stunde unter Rückfluss (Badtemperatur 45°C) gerührt, dann bei 0-10°C mit 20 ml 2N Salzsäure versetzt und dreimal mit Diäthyläther extrahiert. Die organische Phase wurde mit 2N Salzsäure und zweimal mit Wasser gewaschen, über Magnesiumsulfat getrocknet, filtriert und eingedampft. Hierbei wurden 2,5 g [trans-4-[trans-4-(3-Butenyl)cyclohexyl]cyclohexyl]methanol als farblose Kristalle erhalten.

In analoger Weise können folgende Verbindungen hergestellt werden:
[trans-4-(trans-4-Methylcyclohexyl)cyclohexyl]methanol;
[trans-4-(trans-4-Aethylcyclohexyl)cyclohexyl]methanol;
[trans-4-(trans-4-Propylcyclohexyl)cyclohexyl]methanol;
[trans-4-(trans-4-Butylcyclohexyl)cyclohexyl]methanol;
[trans-4-(trans-4-Pentylcyclohexyl)cyclohexyl]methanol;
[trans-4-(trans-4-Hexylcyclohexyl)cyclohexyl]methanol;
[trans-4-(trans-4-Heptylcyclohexyl)cyclohexyl]methanol;
[trans-4-(trans-4-Vinylcyclohexyl)cyclohexyl]methanol;
[trans-4-[trans-4-(1E-Propenyl)cyclohexyl]cyclohexyl]methanol;
[trans-4-[trans-4-(1E-Butenyl)cyclohexyl]cyclohexyl]methanol;
[trans-4-[trans-4-(1E-Pentenyl)cyclohexyl]cyclohexyl]methanol;
[trans-4-[trans-4-(1E-Hexenyl)cyclohexyl]cyclohexyl]methanol;
[trans-4-[trans-4-(1E-Heptenyl)cyclohexyl]cyclohexyl]methanol;
[trans-4-[trans-4-(3E-Pentenyl)cyclohexyl]cyclohexyl]methanol;
[trans-4-[trans-4-(3E-Hexenyl)cyclohexyl]cyclohexyl]methanol;
[trans-4-[trans-4-(3E-Heptenyl)cyclohexyl]cyclohexyl]methanol;
[trans-4-[trans-4-(4-Pentenyl)cyclohexyl]cyclohexyl]methanol;
[trans-4-[trans-4-(4Z-Hexenyl)cyclohexyl]cyclohexyl]methanol;
[trans-4-[trans-4-(4Z-Heptenyl)cyclohexyl]cyclohexyl]methanol;
[trans-4-[2-(trans-4-Methylcyclohexyl)äthyl]cyclohexyl]methanol;
[trans-4-[2-(trans-4-Aethylcyclohexyl)äthyl]cyclohexyl]methanol;
[trans-4-[2-(trans-4-Propylcyclohexyl)äthyl]cyclohexyl]methanol;
[trans-4-[2-(trans-4-Butylcyclohexyl)äthyl]cyclohexyl]methanol;
[trans-4-[2-(trans-4-Pentylcyclohexyl)äthyl]cyclohexyl]methanol;
[trans-4-[2-(trans-4(1E-Propenyl)cyclohexyl)äthyl]cyclohexyl]methanol;
[trans-4-[2-(trans-4-(1E-Butenyl)cyclohexyl)äthyl]cyclohexyl]methanol;
[trans-4-[2-(trans-4-(1E-Pentenyl)cyclohexyl)äthyl]cyclohexyl]methanol;
[trans-4-[2-(trans-4-(3-Butenyl)cyclohexyl)äthyl]cyclohexyl]methanol;
[trans-4-[2-(trans-4-(3E-Pentenyl)cyclohexyl)äthyl]cyclohexyl]methanol;
[trans-4-[2-(trans-4-(4-Pentenyl)cyclohexyl)äthyl]cyclohexyl]methanol.

### Beispiel 2

a) 1,5 g [trans-4-(trans -4-Cyanocyclohexyl)cyclohexyl]methyl-methyläther wurden in 30 ml Eisessig und 15 ml 50-prozentiger Schwefelsäure suspendiert und die Suspension unter Rühren 20 Stunden zum Rückfluss (Badtemperatur 130°C) erhitzt. Anschliessend wurde das Reaktionsgemisch in Methlenchlorid/Wasser verteilt und dreimal mit Methylenchlorid extrahiert. Die organischen Phasen wurden dreimal mit Wasser gewaschen, über Magnesiumsulfat getrocknet, filtriert und eingedampft. Hierbei wurden 1,33 g braune, ölige trans-4-[trans -4-(Methoxymethyl)cyclohexyl]cyclohexancarbonsäure erhalten, welche ohne Reinigung weiter verwendet wurde.
b) Eine Suspension von 390 mg Lithiumaluminiumhydrid in 20 ml Diäthyläther wurde bei Raumtemperatur innert 5 Minuten tropfenweise mit einer Lösung von 1,3 g trans-4-[trans -4-(Methoxymethyl)cyclohexyl]cyclohexancarbonsäure in 40 ml Methylenchlorid versetzt. Das Reaktionsgemisch wurde bei 50°C Badtemperatur 1 Stunde gerührt, dann mit 100 mg Lithiumaluminiumhydrid versetzt und nochmals 1 Stunde gerührt. Anschliessend wurde das Reaktionsgemisch bei ca. 15°C tropfenweise mit 40 ml Wasser versetzt und dreimal mit Methylenchlorid extrahiert. Die organischen Phasen wurden mit verdünnter Salzsäure (pH ca. 1) und zweimal mit Wasser gewaschen, über Magnesiumsulfat getrocknet, filtriert und eingedampft. Chromatographische Reinigung des gelben Rohproduktes (0,96 g) an Kieselgel mit Toluol/Aethylacetat (Vol. 5:1) ergab gelbliches, festes [trans-4-[trans -4-(Methoxymethyl)cyclohexyl]cyclohexyl]methanol.
c) 150 mg Natriumhydrid (als 50-prozentige Suspension in Oel) wird in 10 ml absolutem Tetrahydrofuran suspendiert und dann bei Raumtemperatur innert 3 Minuten tropfenweise mit einer Lösung von 0,5 g [trans-4-[trans -4-(Methoxymethyl)cyclohexyl]cyclohexyl]methanol in 8 ml absolutem Tetrahydrofuran versetzt. Das Gemisch wird 5 Minuten weiter gerührt, dann mit 0,755 g Allylbromid versetzt und 16 Stunden unter Rückfluss (Badtemperatur 70°C) gerührt. Danach wird das Reaktionsgemisch in Diäthyläther/Wasser verteilt und dreimal mit Diäthyläther extrahiert. Die organischen Phasen werden zweimal mit Wasser gewaschen, über Magnesiumsulfat getrocknet, filtriert und eingedampft. Chromatographische Reinigung des erhaltenen Rohproduktes an Kieselgel mit Aethylacetat/Petroläther (Vol. 3:97) ergibt [trans-4-[trans -4-(Methoxymethyl)cyclohexyl]cyclohexyl]methyl-allyläther.

In analoger Weise können folgende Verbindungen hergestellt werden:
[trans-4-[trans-4-(Methoxymethyl)cyclohexyl]cyclohexyl]methyl-2Z-butenyläther;
[trans-4-[trans-4-(Methoxymethyl)cyclohexyl]cyclohexyl]methyl-2Z-pentenyläther;
[trans-4-[trans-4-(Aethoxymethyl)cyclohexyl]cyclohexyl]methyl-allyläther;
[trans-4-[trans-4-(Aethoxymethyl)cyclohexyl]cyclohexyl]methyl-2Z-butenyläther;
[trans-4-[trans-4-(Aethoxymethyl)cyclohexyl]cyclohexyl]methyl-2Z-pentenyläther;
[trans-4-[trans-4-(Propyloxymethyl)cyclohexyl]cyclohexyl]methyl-allyläther;
[trans-4-[trans-4-(Propyloxymethyl)cyclohexyl]cyclohexyl]methyl-2Z-butenyläther;
[trans-4-[trans-4-(Butyloxymethyl)cyclohexyl]cyclohexyl]methyl-allyläther;
[trans-4-[trans-4-(Butyloxymethyl)cyclohexyl]cyclohexyl]methyl-2Z-butenyläther;
[trans-4-[trans-4-(Pentyloxymethyl)cyclohexyl]cyclohexyl]methyl-allyläther;
[trans-4-[trans-4-(Allyloxymethyl)cyclohexyl]cyclohexyl]methyl-allyläther;
[trans-4-[trans-4-(Allyloxymethyl)cyclohexyl]cyclohexyl]methyl-2Z-butenyläther;
[trans-4-[trans-4-(Allyloxymethyl)cyclohexyl]cyclohexyl]methyl-2Z-pentenyläther;
[trans-4-[trans-4-(2Z-Butenyloxymethyl)cyclohexyl]cyclohexyl]methyl-2Z-butenyläther;
[trans-4-[2-(trans-4-(Methoxymethyl)cyclohexyl)äthyl]cyclohexyl]methyl-allyläther;
[trans-4-[2-(trans-4-(Methoxymethyl)cyclohexyl)äthyl]cyclohexyl]methyl-2Z-butenyläther;
[trans-4-[2-(trans-4-(Aethoxymethyl)cyclohexyl)äthyl]cyclohexyl]methyl-allyläther;
[trans-4-[2-(trans-4-(Aethoxymethyl)cyclohexyl)äthyl]cyclohexyl]methyl-2Z-butenyläther;
[trans-4-[2-(trans-4-(Propyloxymethyl)cyclohexyl)äthyl]cyclohexyl]methyl-allyläther;
[trans-4-[2-(trans-4-(Butyloxymethyl)cyclohexyl)äthyl]cyclohexyl]methyl-allyläther;
[trans-4-[2-(trans-4-(Pentyloxymethyl)cyclohexyl)äthyl]cyclohexyl]methyl-allyläther;
[trans-4-[2-(trans-4-(Allyloxymethyl)cyclohexyl)äthyl]cyclohexyl]methyl-allyläther;
[trans-4-[2-(trans-4-(Allyloxymethyl)cyclohexyl)äthyl]cyclohexyl]methyl-2Z-butenyläther;
[trans-4-[2-(trans-4-(2Z-Butenyloxymethyl)cyclohexyl)äthyl]cyclohexyl]methyl-2Z-butenyläther.

### Beispiel 3

In einem Sulfierkolben wurden 290 mg Natriumhydrid als 50-prozentige Suspension in Oel vorgelegt und mit 10 ml Pentan gespült. Dann wurde die überstehende Lösung abgesaugt und der Natriumhydrid-Schlamm innert 2 Minuten tropfenweise mit einer Lösung von 1,0 g [trans-4-[trans-4-(3-Butenyl)cyclohexyl]cyclohexyl]methanol in 15 ml Tetrahydrofuran versetzt. Das Reaktionsgemisch wurde bei Raumtemperatur weitergerührt, nach 5 Minuten mit 0,745 ml Methyljodid versetzt und noch 3 Stunden bei 70°C Badtemperatur weitergerührt. Anschliessend wurde das Reaktionsgemisch in Diäthyläther/Wasser verteilt und dreimal mit Diäthyläther extrahiert. Die organischen Phasen wurden zweimal mit Wasser gewaschen, über Magnesiumsulfat getrocknet, filtriert und eingedampft. Das farblose Rohprodukt (1,1 g) wurde an Kieselgel mit Aethylacetat/Petroläther (Vol. 3:97) chromatographisch gereinigt und aus 40 ml bei -20°C kristallisiert. Hierbei wurden 727 mg farbloser, kristalliner [trans-4-[trans -4-(3-Butenyl)cyclohexyl]cyclohexyl]methyl-methyläther erhalten; Smp. (C-S_{B}) -9,4°C, Phasenübergang (S_{B}-N) 50,3°C, Klp. (N-I) 53,0°C.

In analoger Weise können folgende Verbindungen hergestellt werden:
[trans-4-(trans-4-Vinylcyclohexyl)cyclohexyl]methyl-methyläther, Smp. (C-S_{B}) 5,6°C, Klp. (S_{B}-I) 17,1°C;
[trans-4-[trans-4-(1E-Propenyl)cyclohexyl]cyclohexyl]methyl-methyläther, Smp. (C-S_{B}) 12,3°C, Phasenübergang (S_{B}-N) 22,4°C, Klp. (N-I) 62,2°C;
[trans-4-[trans-4-(1E-Butenyl)cyclohexyl]cyclohexyl]methyl-methyläther, Smp. (C-S_{B}) 6,3°C, Phasenübergang (S_{B}-N) 46,8°C, Klp. (N-I) 49,0°C;
[trans-4-[trans-4-(1E-Pentenyl)cyclohexyl]cyclohexyl]methyl-methyläther;
[trans-4-[trans-4-(1E-Hexenyl)cyclohexyl]cyclohexyl]methyl-methyläther;
[trans-4-[trans-4-(1E-Heptenyl)cyclohexyl]cyclohexyl]methyl-methyläther;
[trans-4-[trans-4-(3E-Pentenyl)cyclohexyl]cyclohexyl]methyl-methyläther, Smp. (C-N) 41°C, Klp. (N-I) 74,3°C;
[trans-4-[trans-4-(3E-Hexenyl)cyclohexyl]cyclohexyl]methyl-methyläther;
[trans-4-[trans-4-(3E-Heptenyl)cyclohexyl]cyclohexyl]methyl-methyläther;
[trans-4-[trans-4-(4-Pentenyl)cyclohexyl]cyclohexyl]methyl-methyläther, Klp. 70,8°C;
[trans-4-[trans-4-(4Z-Hexenyl)cyclohexyl]cyclohexyl]methyl-methyläther;
[trans-4-[trans-4-(4Z-Heptenyl)cyclohexyl]cyclohexyl]methyl-methyläther;
[trans-4-[trans-4-Vinylcyclohexyl]cyclohexyl]methyl-äthyläther, Smp. (C-S_{B}) -19,6°C, Klp. (S_{B}-I) 9,9°C;
[trans-4-[trans-4-(1E-Propenyl)cyclohexyl]cyclohexyl]methyl-äthyläther, Smp. (C-N) 20,8°C, Uebergang (S_{B}-N) 13,3°C, Klp. (N-I) 34,1°C;
[trans-4-[trans-4-(1E-Butenyl)cyclohexyl]cyclohexyl]methyl-äthyläther;
[trans-4-[trans-4-(1E-Pentenyl)cyclohexyl]cyclohexyl]methyl-äthyläther;
[trans-4-[trans-4-(1E-Hexenyl)cyclohexyl]cyclohexyl]methyl-äthyläther;
[trans-4-[trans-4-(1E-Heptenyl)cyclohexyl]cyclohexyl]methyl-äthyläther;
[trans-4-[trans-4-(3-Butenyl)cyclohexyl]cyclohexyl]methyl-äthyläther;
[trans-4-[trans-4-(3E-Pentenyl)cyclohexyl]cyclohexyl]methyl-äthyläther, Smp. (C-S_{B}) 14,3°C, Klp. (S_{B}-I) 57,5°C;
[trans-4-[trans-4-(3E-Hexenyl)cyclohexyl]cyclohexyl]methyl-äthyläther;
[trans-4-[trans-4-(3E-Heptenyl)cyclohexyl]cyclohexyl]methyl-äthyläther;
[trans-4-[trans-4-(4-Pentenyl)cyclohexyl]cyclohexyl]methyl-äthyläther;
[trans-4-[trans-4-(4Z-Hexenyl)cyclohexyl]cyclohexyl]methyl-äthyläther;
[trans-4-[trans-4-(4Z-Heptenyl)cyclohexyl]cyclohexyl]methyl-äthyläther;
[trans-4-[trans-4-Vinylcyclohexyl]cyclohexyl]methyl-propyläther;
[trans-4-[trans-4-(1E-Propenyl)cyclohexyl]cyclohexyl]methyl-propyläther;
[trans-4-[trans-4-(1E-Butenyl)cyclohexyl]cyclohexyl]methyl-propyläther;
[trans-4-[trans-4-(1E-Pentenyl)cyclohexyl]cyclohexyl]methyl-propyläther;
[trans-4-[trans-4-(1E-Hexenyl)cyclohexyl]cyclohexyl]methyl-propyläther;
[trans-4-[trans-4-(1E-Heptenyl)cyclohexyl]cyclohexyl]methyl-propyläther;
[trans-4-[trans-4-(3-Butenyl)cyclohexyl]cyclohexyl]methyl-propyläther;
[trans-4-[trans-4-(3E-Pentenyl)cyclohexyl]cyclohexyl]methyl-propyläther;
[trans-4-[trans-4-(3E-Hexenyl)cyclohexyl]cyclohexyl]methyl-propyläther;
[trans-4-[trans-4-(3E-Heptenyl)cyclohexyl]cyclohexyl]methyl-propyläther;
[trans-4-[trans-4-(4-Pentenyl)cyclohexyl]cyclohexyl]methyl-propyläther;
[trans-4-[trans-4-(4Z-Hexenyl)cyclohexyl]cyclohexyl]methyl-propyläther;
[trans-4-[trans-4-(4Z-Heptenyl)cyclohexyl]cyclohexyl]methyl-propyläther;
[trans-4-(trans-4-Methylcyclohexyl)cyclohexyl]methyl-allyläther;
[trans-4-(trans-4-Aethylcyclohexyl)cyclohexyl]methyl-allyläther;
[trans-4-(trans-4-Propylcyclohexyl)cyclohexyl]methyl-allyläther, Klp. (S_{B}-I) 54°C;
[trans-4-(trans-4-Butylcyclohexyl)cyclohexyl]methyl-allyläther, Smp. (C-S_{B}) -1,4°C, Klp. (S_{B}-I) 72,9°C;
[trans-4-(trans-4-Pentylcyclohexyl)cyclohexyl]methyl-allyläther;
[trans-4-(trans-4-Hexylcyclohexyl)cyclohexyl]methyl-allyläther;
[trans-4-(trans-4-Heptylcyclohexyl)cyclohexyl]methyl-allyläther;
[trans-4-[trans-4-Vinylcyclohexyl)cyclohexyl]methyl-allyläther;
[trans-4-[trans-4-(1E-Propenyl)cyclohexyl]cyclohexyl]methyl-allyläther;
[trans-4-[trans-4-(1E-Butenyl)cyclohexyl]cyclohexyl]methyl-allyläther;
[trans-4-[trans-4-(1E-Pentenyl)cyclohexyl]cyclohexyl]methyl-allyläther;
[trans-4-[trans-4-(1E-Hexenyl)cyclohexyl]cyclohexyl]methyl-allyläther;
[trans-4-[trans-4-(1E-Heptenyl)cyclohexyl]cyclohexyl]methyl-allyläther;
[trans-4-[trans-4-(3-Butenyl)cyclohexyl]cyclohexyl]methyl-allyläther;
[trans-4-[trans-4-(3E-Pentenyl)cyclohexyl]cyclohexyl]methyl-allyläther;
[trans-4-[trans-4-(3E-Hexenyl)cyclohexyl]cyclohexyl]methyl-allyläther;
[trans-4-[trans-4-(3E-Heptenyl)cyclohexyl]cyclohexyl]methyl-allyläther;
[trans-4-[trans-4-(4-Pentenyl)cyclohexyl]cyclohexyl]methyl-allyläther;
[trans-4-[trans-4-(4Z-Hexenyl)cyclohexyl]cyclohexyl]methyl-allyläther;
[trans-4-[trans-4-(4Z-Heptenyl)cyclohexyl]cyclohexyl]methyl-allyläther;
[trans-4-(trans-4-Methylcyclohexyl)cyclohexyl]methyl-2Z-butenyläther;
[trans-4-(trans-4-Aethylcyclohexyl)cyclohexyl]methyl-2Z-butenyläther;
[trans-4-(trans-4-Propylcyclohexyl)cyclohexyl]methyl-2Z-butenyläther;
[trans-4-(trans-4-Butylcyclohexyl)cyclohexyl]methyl-2Z-butenyläther;
[trans-4-(trans-4-Pentylcyclohexyl)cyclohexyl]methyl-2Z-butenyläther;
[trans-4-(trans-4-Hexylcyclohexyl)cyclohexyl]methyl-2Z-butenyläther;
[trans-4-(trans-4-Heptylcyclohexyl)cyclohexyl]methyl-2Z-butenyläther;
[trans-4-[trans-4-Vinylcyclohexyl]cyclohexyl]methyl-2Z-butenyläther;
[trans-4-[trans-4-(1E-Propenyl)cyclohexyl]cyclohexyl]methyl-2Z-butenyläther;
[trans-4-[trans-4-(1E-Butenyl)cyclohexyl]cyclohexyl]methyl-2Z-butenyläther;
[trans-4-[trans-4-(1E-Pentenyl)cyclohexyl]cyclohexyl]methyl-2Z-butenyläther;
[trans-4-[trans-4-(1E-Hexenyl)cyclohexyl]cyclohexyl]methyl-2Z-butenyläther;
[trans-4-[trans-4-(1E-Heptenyl)cyclohexyl]cyclohexyl]methyl-2Z-butenyläther;
[trans-4-[trans-4-(3-Butenyl)cyclohexyl]cyclohexyl]methyl-2Z-butenyläther;
[trans-4-[trans-4-(3E-Pentenyl)cyclohexyl]cyclohexyl]methyl-2Z-butenyläther;
[trans-4-[trans-4-(3E-Hexenyl)cyclohexyl]cyclohexyl]methyl-2Z-butenyläther;
[trans-4-[trans-4-(3E-Heptenyl)cyclohexyl]cyclohexyl]methyl-2Z-butenyläther;
[trans-4-[trans-4-(4-Pentenyl)cyclohexyl]cyclohexyl]methyl-2Z-butenyläther;
[trans-4-[trans-4-(4Z-Hexenyl)cyclohexyl]cyclohexyl]methyl-2Z-butenyläther;
[trans-4-[trans-4-(4Z-Heptenyl)cyclohexyl]cyclohexyl]methyl-2Z-butenyläther;
[trans-4-[2-(trans-4-Vinylcyclohexyl)äthyl]cyclohexyl]methyl-methyläther;
[trans-4-[2-(trans-4-(1E-Propenyl)cyclohexyl)äthyl]cyclohexyl]methyl-methyläther;
[trans-4-[2-(trans-4-(1E-Butenyl)cyclohexyl)äthyl]cyclohexyl)methyl-methyläther;
[trans-4-[2-(trans-4-(1E-Pentenyl)cyclohexyl)äthyl]cyclohexyl]methyl-methyläther;
[trans-4-[2-(trans-4-(3-Butenyl)cyclohexyl)äthyl]cyclohexyl]methyl-methyläther;
[trans-4-[2-(trans-4-(3E-Pentenyl)cyclohexyl)äthyl]cyclohexyl]methyl-methyläther;
[trans-4-[2-(trans-4-(4-Pentenyl)cyclohexyl)äthyl]cyclohexyl]methyl-methyläther;
[trans-4-[2-(trans-4-Vinylcyclohexyl)äthyl]cyclohexyl]methyl-äthyläther;
[trans-4-[2-(trans-4-(1E-Propenyl)cyclohexyl)äthyl]cyclohexyl]methyl-äthyläther;
[trans-4-[2-(trans-4-(1E-Butenyl)cyclohexyl)äthyl]cyclohexyl]methyl-äthyläther;
[trans-4-[2-(trans-4-(1E-Pentenyl)cyclohexyl)äthyl]cyclohexyl]methyl-äthyläther;
[trans-4-[2-(trans-4-(3-Butenyl)cyclohexyl)äthyl]cyclohexyl]methyl-äthyläther;
[trans-4-[2-(trans-4-(3E-Pentenyl)cyclohexyl)äthyl]cyclohexyl]methyl-äthyläther;
[trans-4-[2-(trans-4-(4-Pentenyl)cyclohexyl)äthyl]cyclohexyl]methyl-äthyläther;
[trans-4-[2-(trans-4-Methylcyclohexyl)äthyl]cyclohexyl]methyl-allyläther;
[trans-4-[2-(trans-4-Aethylcyclohexyl)äthyl]cyclohexyl]methyl-allyläther;
[trans-4-[2-(trans-4-Propylcyclohexyl)äthyl]cyclohexyl]methyl-allyläther, Smp. (C-S_{B}) -6,5°C, Klp. (S_{B}-I) 62,4°C;
[trans-4-[2-(trans-4-Butylcyclohexyl)äthyl]cyclohexyl]methyl-allyläther, Smp. (C-S_{B}) -4,1°C, Klp. (S_{B}-I) 76,5°C;
[trans-4-[2-(trans-4-Pentylcyclohexyl)äthyl]cyclohexyl]methyl-allyläther, Smp. (C-S_{B}) -15,9°C, Klp. (S_{B}-I) 83,6°C;
[trans-4-[2-(trans-4-Vinylcyclohexyl)äthyl]cyclohexyl]methyl-allyläther;
[trans-4-[2-(trans-4-(1E-Propenyl)cyclohexyl)äthyl]cyclohexyl]methyl-allyläther;
[trans-4-[2-(trans-4-(1E-Butenyl)cyclohexyl)äthyl]cyclohexyl]methyl-allyläther;
[trans-4-[2-(trans-4-(1E-Pentenyl)cyclohexyl)äthyl]cyclohexyl]methyl-allyläther;
[trans-4-[2-(trans-4-(3-Butenyl)cyclohexyl)äthyl]cyclohexyl]methyl-allyläther;
[trans-4-[2-(trans-4-(3E-Pentenyl)cyclohexyl)äthyl]cyclohexyl]methyl-allyläther;
[trans-4-[2-(trans-4-(4-Pentenyl)cyclohexyl)äthyl]cyclohexyl]methyl-allyläther;
[trans-4-[trans-4-(Methoxymethyl)cyclohexyl]cyclohexyl]methyl-allyläther;
[trans-4-[trans-4-(Methoxymethyl)cyclohexyl]cyclohexyl]methyl-2Z-butenyläther;
[trans-4-[trans-4-(Methoxymethyl)cyclohexyl]cyclohexyl]methyl-2Z-pentenyläther;
[trans-4-[trans-4-(Aethoxymethyl)cyclohexyl]cyclohexyl]methyl-allyläther;
[trans-4-[trans-4-(Aethoxymethyl)cyclohexyl]cyclohexyl]methyl-2Z-butenyläther;
[trans-4-[trans-4-(Aethoxymethyl)cyclohexyl]cyclohexyl]methyl-2Z-pentenyläther;
[trans-4-[trans-4-(Propyloxymethyl)cyclohexyl]cyclohexyl]methyl-allyläther;
[trans-4-[trans-4-(Propyloxymethyl)cyclohexyl]cyclohexyl]methyl-2Z-butenyläther;
[trans-4-[trans-4-(Butyloxymethyl)cyclohexyl]cyclohexyl]methyl-allyläther;
[trans-4-[trans-4-(Butyloxymethyl)cyclohexyl]cyclohexyl]methyl-2Z-butenyläther;
[trans-4-[trans-4-(Pentyloxymethyl)cyclohexyl]cyclohexyl]methyl-allyläther;
[trans-4-[trans-4-(Allyloxymethyl)cyclohexyl]cyclohexyl]methyl-allyläther;
[trans-4-[trans-4-(Allyloxymethyl)cyclohexyl]cyclohexyl]methyl-2Z-butenyläther;
[trans-4-[trans-4-(Allyloxymethyl)cyclohexyl]cyclohexyl]methyl-2Z-pentenyläther;
[trans-4-[trans-4-(2Z-Butenyloxymethyl)cyclohexyl]cyclohexyl]methyl-2Z-butenyläther;
[trans-4-[2-(trans-4-(Methoxymethyl)cyclohexyl)äthyl]cyclohexyl]methyl-allyläther;
[trans-4-[2-(trans-4-(Methoxymethyl)cyclohexyl)äthyl]cyclohexyl]methyl-2Z-butenyläther;
[trans-4-[2-(trans-4-(Aethoxymethyl)cyclohexyl)äthyl]cyclohexyl]methyl-allyläther;
[trans-4-[2-(trans-4-(Aethoxymethyl)cyclohexyl)äthyl]cyclohexyl]methyl-2Z-butenyläther;
[trans-4-[2-(trans-4-(Propyloxymethyl)cyclohexyl)äthyl]cyclohexyl]methyl-allyläther;
[trans-4-[2-(trans-4-(Butyloxymethyl)cyclohexyl)äthyl]cyclohexyl]methyl-allyläther;
[trans-4-[2-(trans-4-(Pentyloxymethyl)cyclohexyl)äthyl]cyclohexyl]methyl-allyläther;
[trans-4-[2-(trans-4-(Allyloxymethyl)cyclohexyl)äthyl]cyclohexyl]methyl-allyläther;
[trans-4-[2-(trans-4-(Allyloxymethyl)cyclohexyl)äthyl]cyclohexyl]methyl-2Z-butenyläther;
[trans-4-[2-(trans-4-(2Z-Butenyloxymethyl)cyclohexyl)äthyl]cyclohexyl]methyl-2Z-butenyläther.

### Beispiel 4

Zur Untersuchung des Einflusses der erfindungsgemässen Verbindungen auf die elektro-optischen Eigenschaften von flüssigkristallinen Gemischen wurden die folgenden binären Mischungen (BM) mit 4-(trans-4-Pentylcyclohexyl)benzonitril hergestellt und mit entsprechenden Gemischen von Alkoxy- bzw. Alkenyloxycyclohexanen verglichen. Die elektro-optischen Daten wurden in einer TN-Zelle mit 8 µm Plattenabstand bei 22°C gemessen; die Ansprechzeiten wurden beim 2,5-fachen Wert von V₁₀ bestimmt. Die entsprechenden Werte für reines 4-(trans-4-Pentylcyclohexyl)benzonitril betragen: Klp. (N-I) 54,6°C, V₁₀ = 1,62V, tₒₙ = 30 ms, t_{off} = 42ms, Δn = 0,120.

### BM-1

50 Gew.-% [trans-4-[trans-4-(3-Butenyl)cyclohexyl]cyclohexyl]methyl-methyläther,
50 Gew.-% 4-(trans-4-Pentylcyclohexyl)benzonitril;
Klp. (N-I) 52,6°C, V₁₀ = 1,73V, tₒₙ = 14ms, t_{off} = 29ms, Δn = 0,085.

### BM-2

50 Gew.-% [trans-4-(trans-4-Vinylcyclohexyl)cyclohexyl]methyl-methyläther,
50 Gew.-% 4-(trans-4-Pentylcyclohexyl)benzonitril;
Klp. (N-I) 38,3°C, V₁₀ = 1,41V, tₒₙ = 14ms, t_{off} = 29ms, Δn = 0,067.

### BM-3

50 Gew.-% [trans-4-[trans-4-(1E-Propenyl)cyclohexyl]cyclohexyl]methyl-methyläther,
50 Gew.-% 4-(trans-4-Pentylcyclohexyl)benzonitril;
Klp. (N-I) 55,9°C, V₁₀ = 1,87V, tₒₙ = 13ms, t_{off} = 25ms, Δn = 0,092.

### BM-4

50 Gew.-% [trans-4-[trans-4-(4-Pentenyl)cyclohexyl]cyclohexyl]methyl-methyläther,
50 Gew.-% 4-(trans-4-Pentylcyclohexyl)benzonitril;
Klp. (N-I) 42,4°C, V₁₀ = 1,41V, tₒₙ = 20ms, t_{off} = 45ms, Δn = 0,074.

### Vergleichsmischung 1

50 Gew.-% trans-4-Allyloxy-1-(trans-4-äthylcyclohexyl)cyclohexan,
50 Gew.-% 4-(trans-4-Pentylcyclohexyl)benzonitril;
Klp. (N-I) 49,9°C, V₁₀ = 1,38V, tₒₙ = 26ms, t_{off} = 42ms, Δn = 0,114.

### Vergleichsmischung 2

50 Gew.-% trans-4-Aethoxy-1-[trans-4-(3-butenyl)cyclohexyl)cyclohexan,
50 Gew.-% 4-(trans-4-Pentylcyclohexyl)benzonitril;
Klp. (N-I) 44,4°C, V₁₀ = 1,66V, tₒₙ = 21ms, t_{off} = 31ms, Δn = 0,082.

### Beispiel 5

Die nematischen Mischungen der in den Tabellen 1 und 3 angegebenen Zusammensetzungen illustrieren die Verwendung der erfindungsgemässen Verbindungen in komplexeren Flüssigkristallgemischen. Die Konzentrationsangaben in den Tabellen 1 und 3 sind in Gewichtsprozenten angegeben. Die physikalischen Daten der Gemische sind in den Tabellen 2 und 4 angegeben. Die elektro-optischen Daten wurden in einer TN-Zelle mit 6 µm (für Mischungen M-1 bis M-4 in Tabelle 2) bzw. 8 µm (für Mischungen M-5 bis M-8 in Tabelle 4) Plattenabstand bei 22°C gemessen, wobei die Ansprechzeiten bei einer Spannung entsprechend dem 2,5-fachen Wert von V₁₀ bestimmt wurden. M-8 ist eine Vergleichsmischung zu M-7 ohne Verbindung der Formel I. Die in den Tabellen 1 und 3 für die Komponenten verwendeten Bezeichnungen besitzen folgende Bedeutungen:
- 2P(1)P2 =: 5-Aethyl-2-(4-äthylphenyl)pyrimidin,
- 0(3)P(1)P2 =: 5-(3-Butenyl)-2-(4-äthylphenyl)pyrimidin,
- 0(4)P(1)P2 =: 5-(4-Pentenyl)-2-(4-äthylphenyl)pyrimidin,
- 0(3)P(1)PF =: 5-(3-Butenyl)-2-(4-fluorphenyl)pyrimidin,
- 3CPO2 =: 4-Aethoxy-1-(trans-4-propylcyclohexyl)benzol,
- 3CP =: 4-(trans-4-Propylcyclohexyl)benzonitril,
- 0d(1)CP =: 4-(trans-4-Vinylcyclohexyl)benzonitril,
- 1d(1)CP =: 4-[trans-4-(1E-Propenyl)cyclohexyl]benzonitril,
- 2d(1)CP =: 4-[trans-4-(1E-Butenyl)cyclohexyl]benzonitril,
- 0(3)CP =: 4-[trans-4-(3-Butenyl)cyclohexyl]benzonitril,
- 3CPS =: 4-(trans-4-Propylcyclohexyl)phenylisothiocyanat,
- 1d(3)CCO1 =: trans-4-Methoxy-1-[trans-4-(3E-pentenyl)cyclohexyl]cyclohexan,
- 0d(3)CCO2 =: trans-4-Aethoxy-1-[trans-4-(3-butenyl)cyclohexyl]cyclohexan,
- 1d(3)CCO2 =: trans-4-Aethoxy-1-[trans-4-(3E-pentenyl)cyclohexyl]cyclohexan,
- 0d(4)CCO2 =: trans-4-Aethoxy-1-[trans-4-(4-pentenyl)cyclohexyl]cyclohexan,
- 3CAPO2 =: 4-Aethoxy-1-[2-(trans-4-propylcyclohexyl)äthyl]benzol,
- 2CP(1)P5 =: 5-(trans-4-Aethylcyclohexyl)-2-(4-pentylphenyl)pyrimidin,
- 1d(3)CPP2 =: 4′-Aethyl-4-[trans-4-(3E-pentenyl)cyclohexyl]biphenyl,
- 1d(3)CPP3 =: 4′-Propyl-4-[trans-4-(3E-pentenyl)cyclohexyl]biphenyl,
- 1d(1)CCP1 =: 4-Methyl-1-[trans-4-(trans-4-(1E-propenyl)cyclohexyl)cyclohexyl]benzol,
- 3CCPF =: 4-Fluor-1-[trans-4-(trans-4-propylcyclohexyl)cyclohexyl]benzol,
- 0d(1)CCPF =: 4-Fluor-1-[trans-4-(trans-4-vinylcyclohexyl)cyclohexyl]benzol,
- 1d(1)CCPF =: 4-Fluor-1-[trans-4-(trans-4-(1E-propenyl)cyclohexyl)cyclohexyl]benzol,
- 0d(3)CCPF =: 4-Fluor-1-[trans-4-(trans-4-(3-butenyl)cyclohexyl)cyclohexyl]benzol,
- 3d(1)CPC3 =: 4-(trans-4-Propylcyclohexyl)-1-[trans-4-(1E-pentenyl)cyclohexyl]benzol,
- 5CPAC4 =: 4-(trans-5-Pentylcyclohexyl)-1-[2-(trans-4-butylcyclohexyl)äthyl]benzol,
- 0d(3)CCEPF =: trans-4-[trans-4-(3-Butenyl)cyclohexyl]cyclohexancarbonsäure-4-fluorphenylester,
- 3CEPCd(3)1 =: trans-4-Propylcyclohexancarbonsäure-4-[trans-4-(3E-pentenyl)cyclohexyl]phenylester,
- 3CPPC3 =: 4,4′-Bis(trans-4-propylcyclohexyl)biphenyl,
- 5CP(1)PC3 =: 5-(trans-4-Pentylcyclohexyl)-2-[4-(trans-4-propylcyclohexyl)phenyl]pyrimidin,
- 5CPPAC4 =: 4′-(trans-4-Pentylcyclohexyl)-4-[2-(trans-4-butylcyclohexyl)äthyl]biphenyl,
- 0d(1)CC101 =: [trans-4-(trans-4-Vinylcyclohexyl)cyclohexyl]methyl-methyläther,
- 1d(1)CC101 =: [trans-4-[trans-4-(1E-Propenyl)cyclohexyl]cyclohexyl]methyl-methyläther,
- 0d(3)CC101 =: [trans-4-[trans-4-(3-Butenyl)cyclohexyl]cyclohexyl]methyl-methyläther,
- 3CC1Od(4)0 =: [trans-4-(trans-4-Propylcyclohexyl)cyclohexyl]methyl-allyläther.

**Tabelle 1**

| Konzentrationsangaben in Gew.-% | | | | |
|---|---|---|---|---|
| Mischung | M-1 | M-2 | M-3 | M-4 |
| 3CP02 | 6% | 6% | 6% | - |
| 3CP | 10% | 10% | 10% | 10% |
| 0d(1)CP | 7% | 7% | 7% | 7% |
| 1d(1)CP | 8% | 8% | 8% | 8% |
| 1d(3)CC01 | 10% | 10% | 10% | 10% |
| 0d(3)CC02 | 10% | 10% | 10% | 10% |
| 0d(4)CC02 | - | - | - | 6% |
| 1d(1)CCP1 | - | - | - | 6% |
| 3CCPF | 8% | 8% | 8% | - |
| 0d(1)CCPF | - | - | - | 5% |
| 1d(1)CCPF | - | - | - | 5% |
| 3d(1)CPC3 | 8% | 8% | 8% | - |
| 5CPAC4 | 8% | 8% | 8% | 8% |
| 3CEPCd(3)1 | 6% | 6% | 6% | 6% |
| 3CPPC3 | 4% | 4% | 4% | - |
| 5CP(1)PC3 | 3% | 3% | 3% | 3% |
| 5CPPAC4 | - | - | - | 4% |
| 0d(1)CC101 | 12% | - | - | - |
| 1d(1)CC101 | - | - | - | 12% |
| 0d(3)CC101 | - | - | 12% | - |
| 3CC10d(4)0 | - | 12% | - | - |

**Tabelle 2**

| Mischung | | M-1 | M-2 | M-3 | M-4 |
|---|---|---|---|---|---|
| V₁₀ | | 2,15V | 2,20V | 2,28V | 2,30V |
| tₒₙ | (22°C) | 11ms | 13ms | 11ms | 11ms |
| | (-20°C) | 157ms | 174ms | 165ms | 178ms |
| | (-30°C) | 416ms | 425ms | 450ms | 497ms |
| t_{off} | (22°C) | 22ms | 23ms | 20ms | 20ms |
| | (-20°C) | 229ms | 253ms | 244ms | 256ms |
| | (-30°C) | 565ms | 609ms | 596ms | 657ms |
| Smp. | | <-40°C | <-40°C | <-40°C | <-30°C |
| Klp. | (N-I) | 85°C | 85°C | 89°C | 93°C |
| Δn | | 0,098 | 0,095 | 0,099 | 0,090 |

**Tabelle 3**

| Konzentrationsangaben in Gew.-% | | | | |
|---|---|---|---|---|
| Mischung | M-5 | M-6 | M-7 | M-8 |
| 2P(1)P2 | | 8% | 8,00% | 8,00% |
| 0d(3)P(1)P2 | 6% | | | |
| 0d(4)P(1)P2 | 5% | | | |
| 0d(3)P(1)PF | 5% | | | |
| 3CP | 7% | 6% | 5,88% | 6,44% |
| 1d(1)CP | 6% | 10% | 9,24% | 10,12% |
| 2d(1)CP | 9% | | | |
| 0d(3)CP | | 7% | 6,72% | 7,36% |
| 3CPS | 8% | 9% | 7,56% | 8,28% |
| 1d(3)CCO2 | | | 8,40% | 9,20% |
| 3CAPO2 | | 7% | 8,40% | 9,20% |
| 2CP(1)P5 | 4% | | | |
| 1d(3)CPP2 | 5% | 5% | 4,20% | 4,60% |
| 1d(3)CPP3 | 6% | 6% | 5,04% | 5,52% |
| 1d(1)CCP1 | 6% | | | |
| 1d(1)CCPF | 5% | 4% | 3,36% | 3,68% |
| 0d(3)CCPF | 5% | | | |
| 0d(3)CCEPF | 6% | 8% | 6,72% | 7,36% |
| 3CEPCd(3)1 | 5% | 4% | 4,20% | 4,60% |
| 5CPAC4 | | 8% | 7,56% | 8,28% |
| 5CP(1)PC3 | 2% | 3% | 2,52% | 2,76% |
| 5CPPAC4 | | 5% | 4,20% | 4,60% |
| 1d(1)CC101 | 10% | 10% | 8,00% | |

**Tabelle 4**

| Mischung | | M-5 | M-6 | M-7 | M-8 |
|---|---|---|---|---|---|
| V₁₀ | | 2,18V | 2,42V | 2,43V | 2,4V |
| tₒₙ | (22°C) | 16ms | 19ms | 17ms | 18ms |
| | (-20°C) | 287ms | 322ms | 281ms | 301ms |
| | (-30°C) | 888ms | 984ms | 830ms | 931ms |
| t_{off} | (22°C) | 29ms | 32ms | 30ms | 31ms |
| | (-20°C) | 419ms | 477ms | 397ms | 424ms |
| | (-30°C) | 1270ms | 1441ms | 1120ms | 1275ms |
| Smp. | | <-30°C | <-30°C | <-30°C | <-30°C |
| Klp. | (N-I) | 91°C | 96°C | 89°C | 93°C |
| Δn | | 0,139 | 0,135 | 0,127 | 0,133 |

## Patentansprüche

1. Verbindungen der allgemeinen Formel 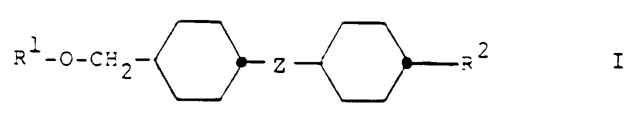 worin Z eine einfache Kovalenzbindung oder -CH₂CH₂-darstellt; R¹ Alkyl oder 2-Alkenyl bezeichnet; und R² 1E-Alkenyl, 3E-Alkenyl, 4-Alkenyl, (2-Alkenyl)oxymethyl oder, wenn R¹ 2-Alkenyl bezeichnet und Z -CH₂CH₂- darstellt, auch Alkyl oder Alkoxymethyl bedeutet.

2. Verbindungen nach Anspruch 1, dadurch gekennzeichnet, dass R¹ und R² geradkettige Reste bedeuten.

3. Verbindungen nach Anspruch 1 oder 2, dadurch gekennzeichnet, dass R¹ höchstens 10, vorzugsweise höchstens 5 Kohlenstoffatome aufweist.

4. Verbindungen nach einem der Ansprüche 1 bis 3, dadurch gekennzeichnet, dass R² höchstens 12, vorzugsweise höchtens 7 Kohlenstoffatome aufweist.

5. Verbindungen nach einem der Ansprüche 1 bis 4, dadurch gekennzeichnet, dass R¹ für 2-Alkenyl und/oder R² für 1E-Alkenyl, 3E-Alkenyl, 4-Alkenyl oder (2-Alkenyl)oxymethyl steht.

6. Flüssigkristallines Gemisch mit mindestens 2 Komponenten, dadurch gekennzeichnet, dass mindestens eine Komponente eine Verbindung der in Anspruch 1 definierten Formel I ist.

7. Flüssigkristallines Gemisch nach Anspruch 6, dadurch gekennzeichnet, dass es eine oder mehrere Verbindungen der in Anspruch 1 definierten Formel I und eine oder mehrere Verbindungen mit positiver dielektrischer Anisotropie enthält.

8. Flüssigkristallines Gemisch nach Anspruch 6 oder 7, dadurch gekennzeichnet, dass es eine oder mehrere Verbindungen der Formel I und eine oder mehrere Verbindungen aus der Gruppe der Verbindungen der allgemeinen Formeln 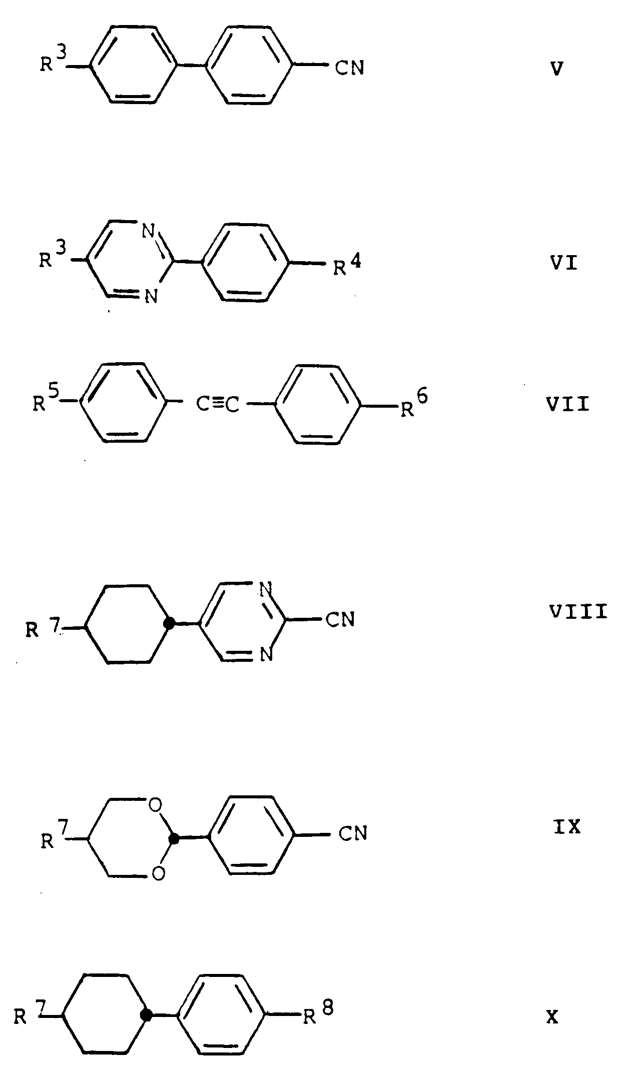 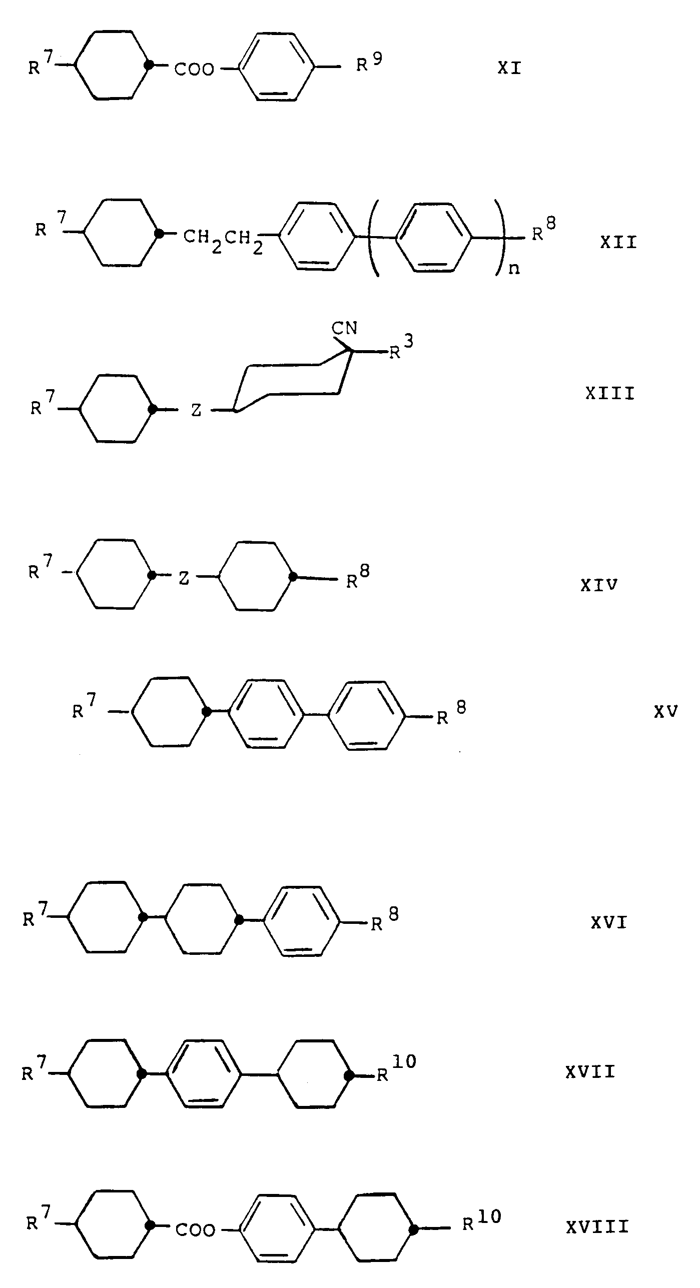 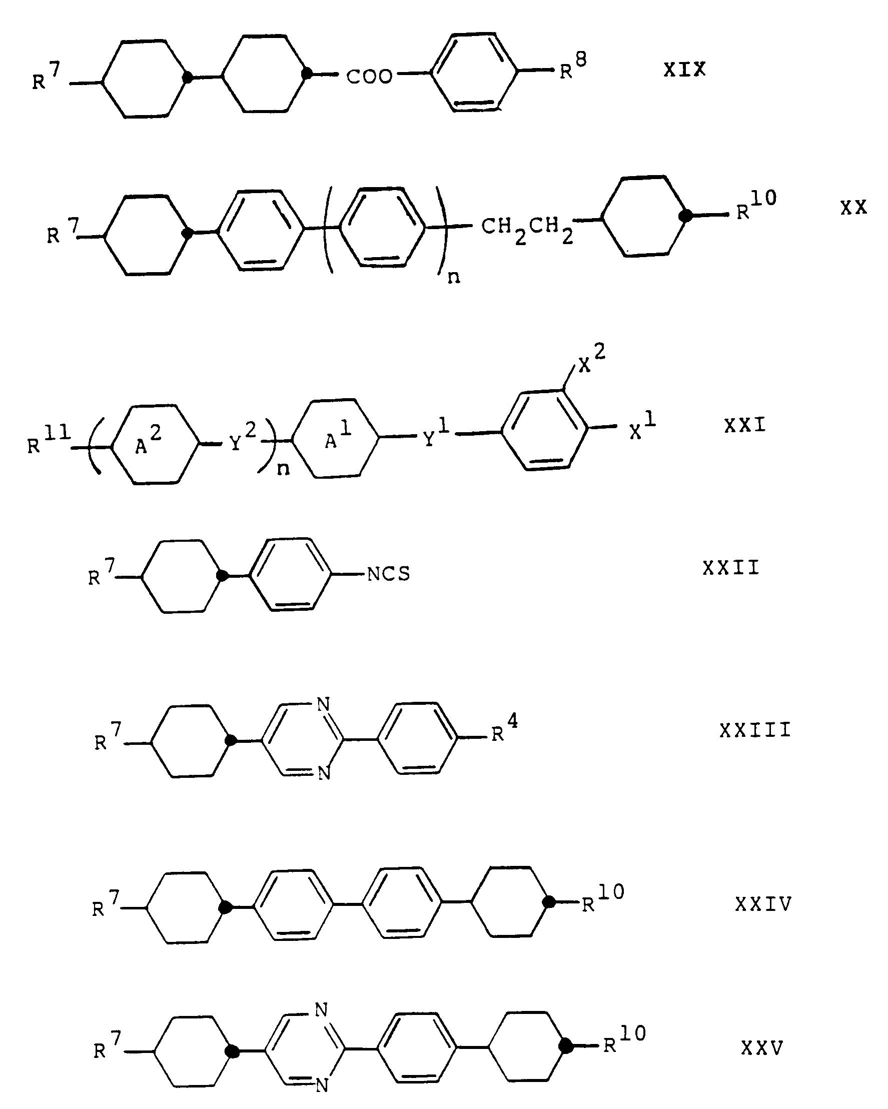 worin R³ Alkyl, 3E-Alkenyl oder 4-Alkenyl bedeutet; R⁴ Alkyl, Cyano oder Fluor darstellt; R⁵ und R⁶ Alkyl oder Alkoxy bezeichnen; R⁷ und R¹⁰ unabhängig voneinander Alkyl, 1E-Alkenyl, 3E-Alkenyl oder 4-Alkenyl bedeuten; R⁸ Cyano, Alkyl, 1E-Alkenyl, 3E-Alkenyl, 4-Alkenyl, Alkoxy, 2E-Alkenyloxy oder 3-Alkenyloxy bezeichnet; R⁹ Alkoxy, 2E-Alkenyloxy oder 3-Alkenyloxy bedeutet; n für die Zahl 0 oder 1 steht; Z eine einfache Kovalenzbindung oder -CH₂CH₂- darstellt; X¹ Fluor oder Chlor und X² Wasserstoff, Fluor oder Chlor bezeichnen; R¹¹ Alkyl, 3E-Alkenyl, 4-Alkenyl, Alkoxy, 2E-Alkenyloxy oder 3-Alkenyloxy bedeutet; eine der Gruppen Y¹ und Y² eine einfache Kovalenzbindung, -COO-, -OOC-, -CH₂CH₂-, -CH₂O- oder -OCH₂ - und die andere der Gruppen Y¹ und Y² eine einfache Kovalenzbindung bedeutet; und die Ringe A¹ und A² unabhängig voneinander substituiertes oder unsubstituiertes trans-1,4-Cyclohexylen, in welchem gegebenenfalls 2 nicht benachbarte CH₂-Gruppen durch Sauerstoff ersetzt sind, oder substituiertes oder unsubstituiertes 1,4-Phenylen, in welchem gegebenenfalls 1 oder 2 CH₂-Gruppen durch Stickstoff ersetzt sind, darstellen,
enthält.

9. Flüssigkristallines Gemisch nach einem der Ansprüche 6 bis 8, dadurch gekennzeichnet, dass der Anteil an Verbindungen der Formel I im Gesamtgemisch 1-70 Gew.-%, vorzugsweise 3-40 Gew.-% beträgt.

10. Verfahren zur Herstellung der Verbindungen der allgemeinen Formel 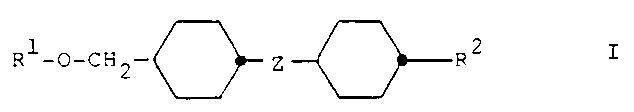 worin Z eine einfache Kovalenzbindung oder -CH₂CH₂-darstellt; R¹ Alkyl oder 2-Alkenyl bezeichnet; und R² 1E-Alkenyl, 3E-Alkenyl, 4-Alkenyl, (2-Alkenyl)oxymethyl oder, wenn R¹ 2-Alkenyl bezeichnet und/oder Z -CH₂CH₂- darstellt, auch Alkyl oder Alkoxymethyl bedeutet,
dadurch gekennzeichnet, dass man eine Verbindung der allgemeinen Formel 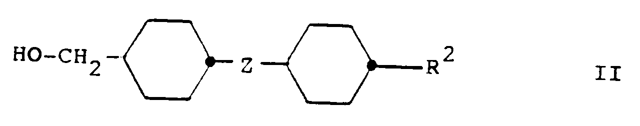 mit einer Verbindung der allgemeinen Formel
R¹-X III
worin X Chlor, Brom oder Jod bezeichnet, und R¹ und R² die obigen Bedeutungen haben,
veräthert.

## Claims

1. Compounds of the general formula 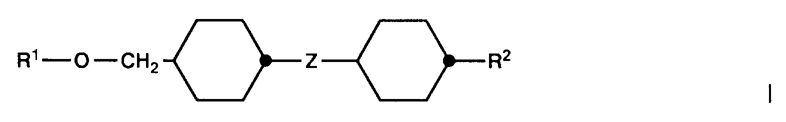 wherein Z represents a single covalent bond or -CH₂-CH₂-; R¹ denotes alkyl or 2-alkenyl; and R² signifies 1E-alkenyl, 3E-alkenyl, 4-alkenyl, (2-alkenyl)oxymethyl or, when R¹ denotes 2-alkenyl and Z represents -CH₂-CH₂-, also alkyl or alkoxymethyl.

2. Compounds according to claim 1, characterized in that R¹ and R² signify straight-chain residues.

3. Compounds according to claim 1 or 2, characterized in that R¹ has a maximum of 10, preferably a maximum of 5, carbon atoms.

4. Compounds according to any one of claims 1 to 3, characterized in that R² has a maximum of 12, preferably a maximum of 7, carbon atoms.

5. Compounds according to any one of claims 1 to 4, characterized in that R¹ stands for 2-alkenyl and/or R² stands for 1E-alkenyl, 3E-alkenyl, 4-alkenyl or (2-alkenyl)oxymethyl.

6. A liquid crystalline mixture having at least 2 components, characterized in that at least one component is a compound of formula I defined in claim 1.

7. A liquid crystalline mixture according to claim 6, characterized in that it contains one or more compounds of formula I defined in claim 1 and one or more compounds having positive dielectric anisotropy.

8. A liquid crystalline mixture according to claim 6 or 7, characterized in that it contains one or more compounds of formula I and one or more compounds from the group of compounds of the general formulae 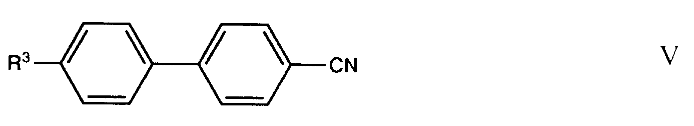 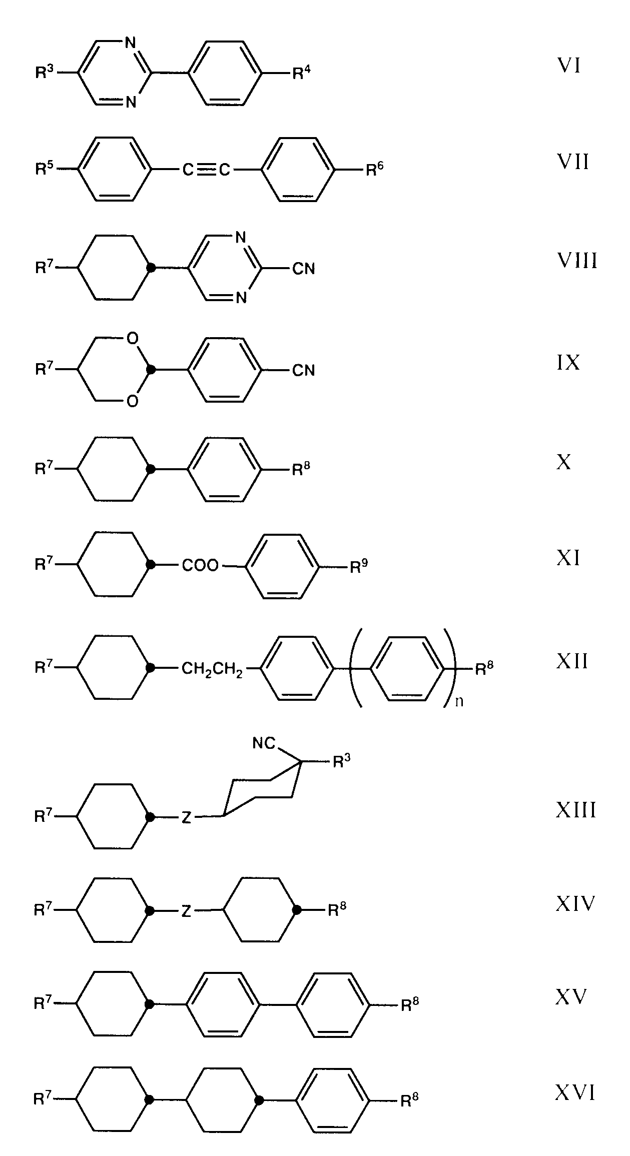 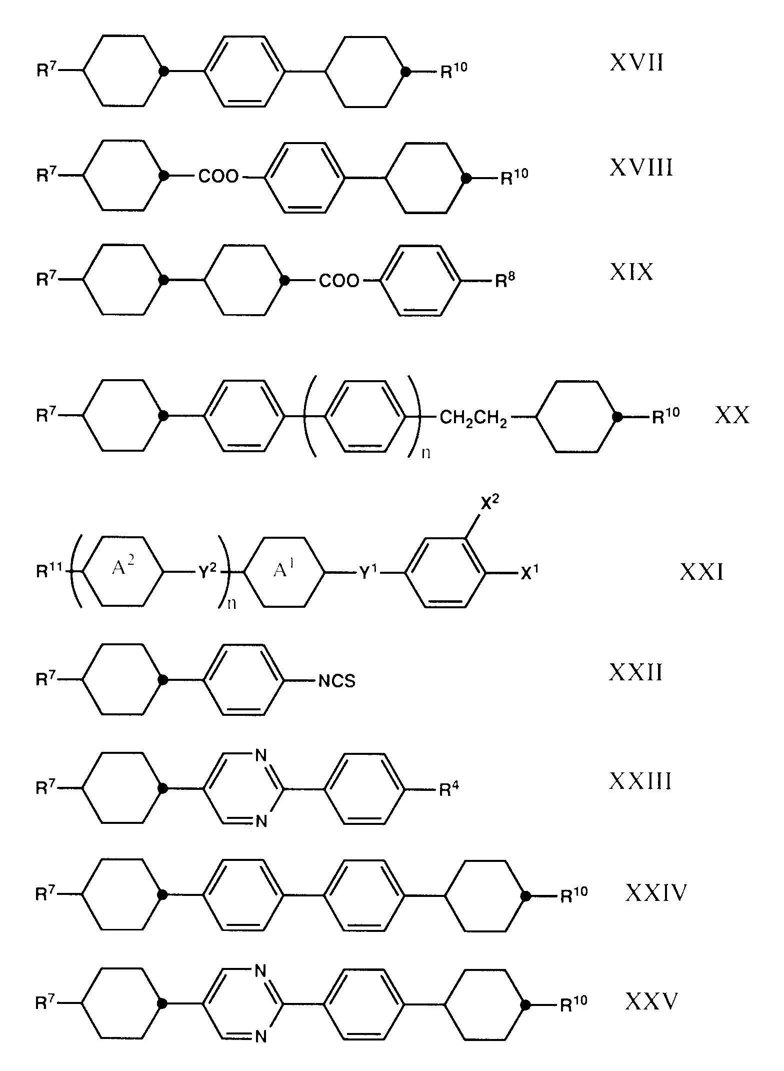 wherein R³ signifies alkyl, 3E-alkenyl or 4-alkenyl; R⁴ represents alkyl, cyano or fluorine; R⁵ and R⁶ denote alkyl or alkoxy; R⁷ and R¹⁰ each independently signify alkyl, 1E-alkenyl, 3E-alkenyl or 4-alkenyl; R⁸ denotes cyano, alkyl, 1E-alkenyl, 3E-alkenyl, 4-alkenyl, alkoxy, 2E-alkenyloxy or 3-alkenyloxy; R⁹ signifies alkoxy, 2E-alkenyloxy or 3-alkenyloxy; n stands for the number O or 1; Z represents a single covalent bond or -CH₂CH₂-; X¹ denotes fluorine or chlorine and X² denotes hydrogen, fluorine or chlorine; R¹¹ signifies alkyl, 3E-alkenyl, 4-alkenyl, alkoxy, 2E-alkenyloxy or 3-alkenyloxy; one of the groups Y¹ and Y² signifies a single covalent bond, -COO-, -OOC-, -CH₂CH₂-, -CH₂O- or -OCH₂- and the other of the groups Y¹ and Y² signifies a single covalent bond; and rings A¹ and A² each independently represent substituted or unsubstituted trans-1,4-cyclohexylene, in which optionally 2 non-adjacent CH₂ groups are replaced by oxygen, or substituted or unsubstituted 1,4-phenylene, in which optionally 1 or 2 CH groups is/are replaced by nitrogen.

9. A liquid crystalline mixture according to any one of claims 6 to 8, characterized in that the content of compounds of formula I in the final mixture amounts to 1-70 wt.%, preferably 3-40 wt.%.

10. A process for the manufacture of the compounds of the general formula 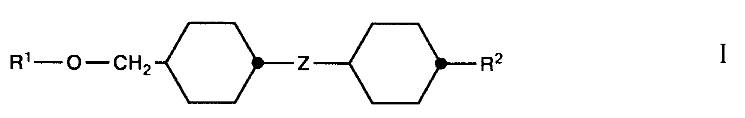 wherein Z represents a single covalent bond or -CH₂-CH₂-; R¹ denotes alkyl or 2-alkenyl and R² signifies 1E-alkenyl, 3E-alkenyl, 4-alkenyl, (2-alkenyl)oxymethyl or, when R¹ denotes 2-alkenyl and/or Z represents -CH₂-CH₂-, also alkyl or alkoxymethyl,
characterized by etherifying a compound of the general formula 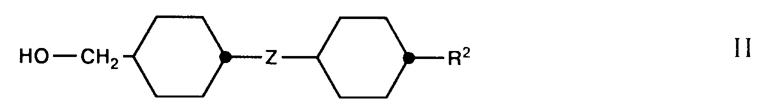 with a compound of the general formula
R¹-X III
wherein X denotes chlorine, bromine or iodine and R¹ and R² have the above significances.

## Revendications

1. Composés de formule générale 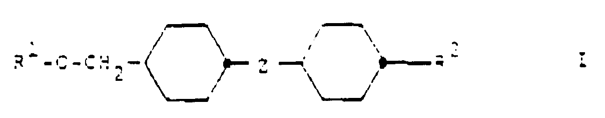 dans laquelle Z représente une liaison de covalence simple ou -CH₂CH₂- ; R¹ représente un alcoyle ou un 2-alcényle; et R² représente un 1E-alcényle, un 3E-alcényle, un 4-alcényle, un (2-alcényl)oxyméthyle ou encore, lorsque R¹ représente un 2-alcényle et Z représente -CH₂CH₂- , un alcoyle ou un alcoxyméthyle.

2. Composés selon la revendication 1, caractérisés en ce que R¹ et R² représentent des radicaux à chaîne droite.

3. Composés selon la revendication 1 ou 2, caractérisés en ce que R¹ présente au plus 10, de préférence au plus 5, atomes de carbone.

4. Composés selon l'une des revendications 1 à 3, caractérisés en ce que R² présente au plus 12, de préférence au plus 7, atomes de carbone.

5. Composés selon l'une des revendications 1 à 4, caractérisés en ce que R¹ représente un 2-alcényle et/ou R² un 1E-alcényle, un 3E-alcényle, un 4-alcényle ou un (2-alcényl)-oxyméthyle.

6. Mélange à cristaux liquides avec au moins 2 composants, caractérisé en ce qu'au moins un composant est un composé de formule I définie dans la revendication 1.

7. Mélange à cristaux liquides selon la revendication 6, caractérisé en ce qu'il contient un ou plusieurs composés de formule I définie dans la revendication 1 et un ou plusieurs composés a anisotropie diélectrique positive.

8. Mélange à cristaux liquides selon la revendication 6 ou 7, caractérisé en ce qu'il contient un ou plusieurs composés de formule I et un ou plusieurs composés du groupe des composés de formules générales 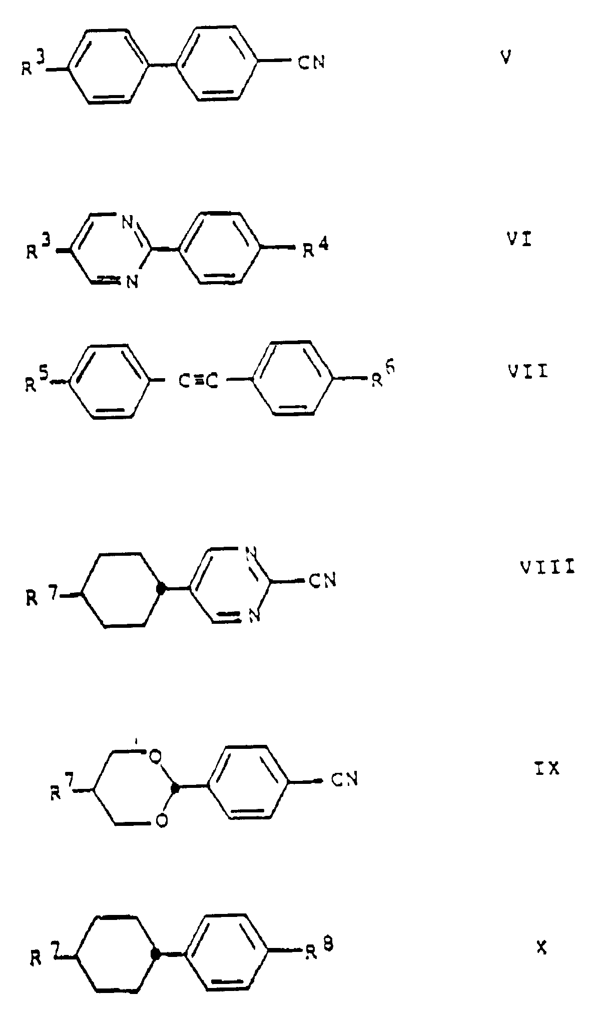 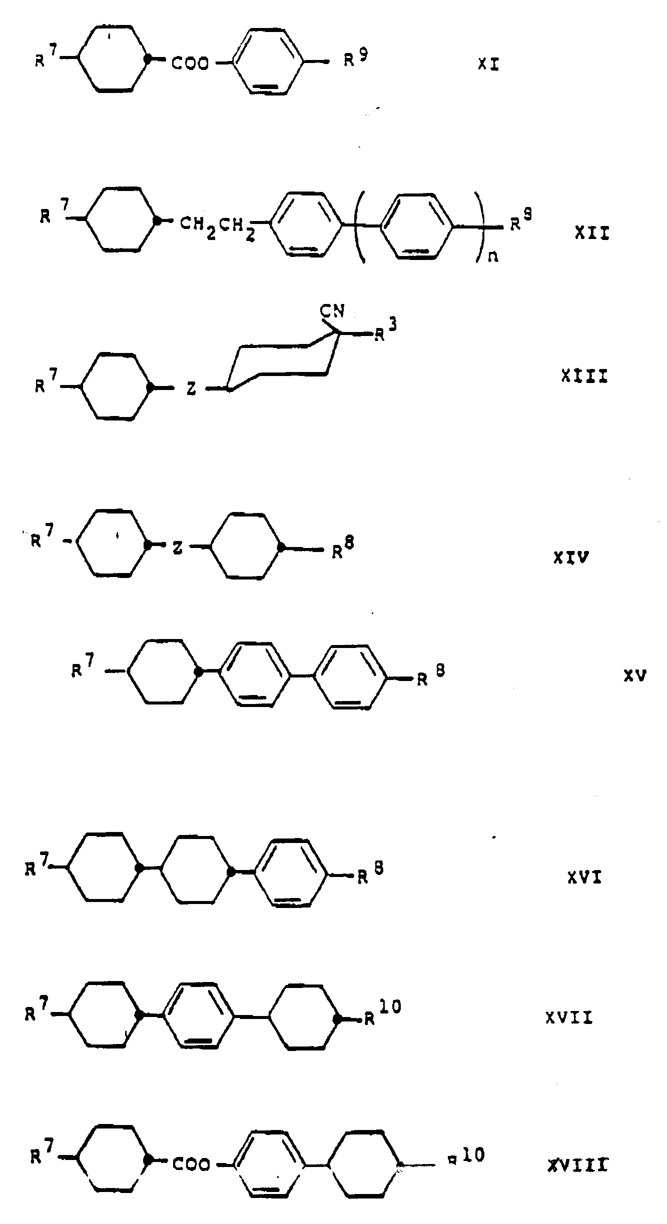 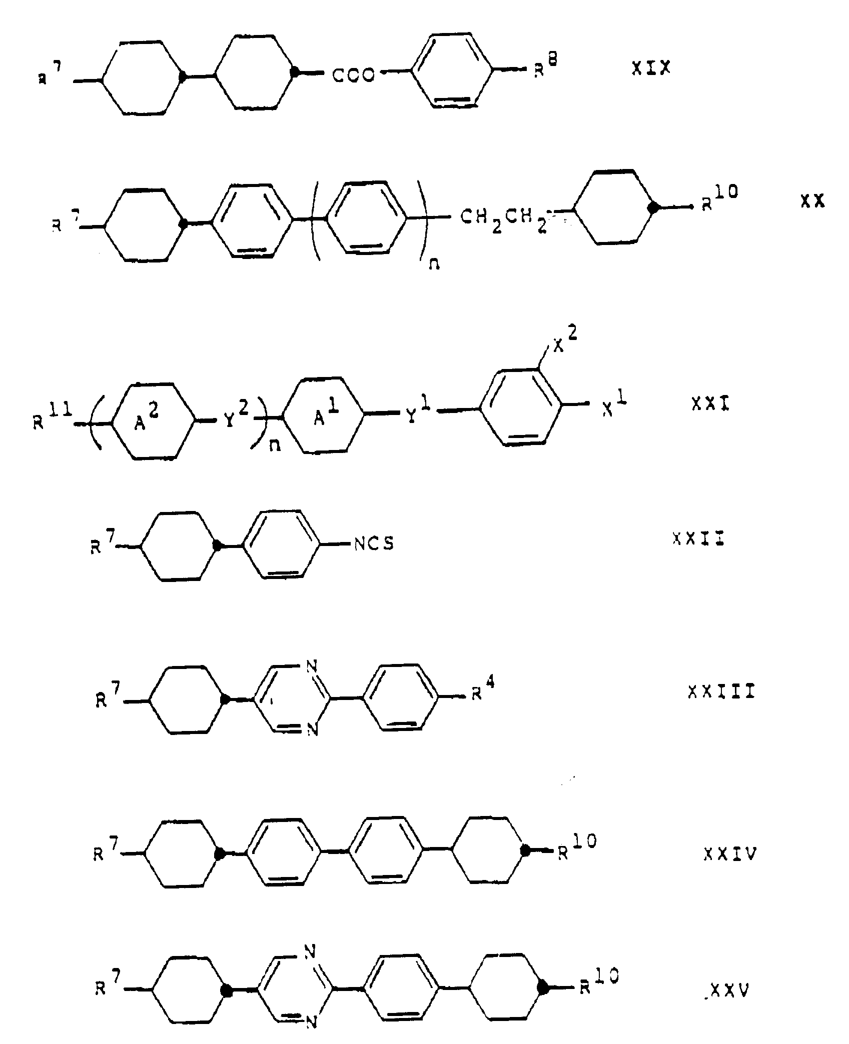 dans lesquelles R³ représente un alcoyle, un 3E-alcényle ou un 4-alcényle; R⁴ représente un alcoyle, un cyano ou un fluor; R⁵ et R⁶ représentent un alcoyle ou un alcoxy; R⁷ et R¹⁰ représentent indépendamment l'un de l'autre un alcoyle, un 1E-alcényle, un 3E-alcényle ou un 4-alcényle; R⁸ représente un cyano, un alcoyle, un 1E-alcényle, un 3E-alcényle, un 4-alcényle, un alcoxy, un 2E-alcényloxy ou un 3-alcényloxy; R⁹ représente un alcoxy, un 2E-alccényloxy ou un 3-alcényloxy; n représente le nombre 0 ou 1; Z représente une liaison de covalence simple ou -CH₂CH₂- ; X¹ représente un fluor ou un chlore et X² représente un hydrogène, un fluor ou un chlore; R¹¹ représente un alcoyle, un 3E-alcényle, un 4-alcényle, un alcoxy, un 2E-alcényloxy ou un 3-alcényloxy; l'un des groupes Y¹ et Y² représente une liaison de covalence simple, -COO- , -OOC- , -CH₂CH₂- , -CH₂O- ou -OCH₂- et les autres groupes Y¹ et Y² représentent une liaison de covalence simple; et les noyaux A¹ et A² représentent indépendamment l'un de l'autre un trans-1,4-cyclohexylène substitué ou non substitué, dans lequel le cas échéant deux groupes CH₂ sont remplacés par de l'oxygène, ou un 1,4-phénylène substitué ou non substitué dans lequel le cas échéant 1 ou 2 groupes CH₂ sont remplacés par de l'oxygène.

9. Mélange à cristaux liquides selon l'une des revendications 6 à 8, caractérisé en ce que la proportion de composés de formule I dans le mélange total s'élève à 1-70% en poids, de préférence 3-40% en poids.

10. Procédé de préparation des composés de formule générale 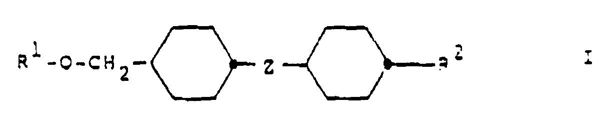 dans laquelle Z représente une liaison de covalence simple ou -CH₂CH₂- ; R¹ représente un alcoyle ou un 2-alcényle; et R² représente un 1E-alcényle, un 3E-alcényle, un 4-alcényle, un (2-alcényl)oxyméthyle ou encore, lorsque R¹ représente un 2-alcényle et/ou Z représente CH₂CH₂ , un alcoyle ou un alcoxyméthyle, caractérisé en ce qu'on éthérifie un composé de formule générale 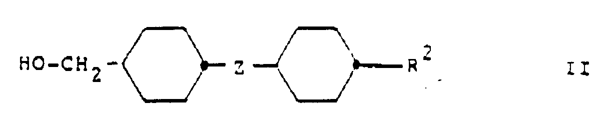 avec un composé de formule générale
R¹ - X III
dans laquelle X représente un chlore, un brome ou un iode, et R¹ et R² ont les significations données ci-dessus.
